# EUROPEAN PATENT SPECIFICATION

(11) **EP 3 571 992 B1**
(45) Date of publication and mention of the grant of the patent: **16.10.2024**
(21) Application number: 19170427.9
(22) Date of filing: 19.04.2019
(51) Int. Cl.: A61B 5/11, A61F 5/01

(54) **MITIGATING EFFECTS OF NEURO-MUSCULAR AILMENTS**
ABSCHWÄCHUNG DER AUSWIRKUNGEN VON NEUROMUSKULÄREN BESCHWERDEN
ATTÉNUATION DES EFFETS DE MALADIES NEUROMUSCULAIRES

(30) Priority: 23.05.2018 US 201815987593
(43) Date of publication of application: 27.11.2019
(73) Proprietor: INTEL Corporation, Santa Clara, CA 95054 (US)
(72) Inventor: KRIMON, Yuri, Folsom, CA California 95630 (US); MIDDELA, Shirisha, Folsom, CA California 95630 (US); BARTFAI-WALCOTT, Katalin, El Dorado Hills, CA California 95762 (US); MURPHY, Ingrid, El Dorado Hills, CA California 95762 (US); CHIVUKULA, Vamsee Vardhan, Folsom, CA California 95630 (US); IMHOFF, Michael, Folsom, CA California 95630 (US); ONIYINDE, Olugbemisola, Folsom, CA California 95630 (US)
(74) Representative: Rummler, Felix

(56) References cited:
- WO-A1-2017/062755
- GB-A- 2 444 393
- US-A1- 2005 171 577
- US-A1- 2017 014 625
- US-B1- 9 314 190

## Description

### TECHNICAL FIELD

An embodiment of the present subject matter relates generally to assistive devices, and, more specifically but not limited to, a device that mitigates the effects of neuro-muscular ailments such as tremors or loss of strength through inferencing or predicting intended motion.

### BACKGROUND

Millions of people suffer from degenerative motor control (e.g., Parkinson's disease, multiple sclerosis, etc.), which inhibit one's ability to perform essential tasks such as eating, getting dressed, writing, etc. Many suffer from tremors in the upper extremities. Loss of strength, flexibility, and control occurs in upper and lower extremities as well. Shaking, tremors, and loss of muscle strength may be a result of various neurological disorders. The underlying causes of essential tremors are unclear and no effective treatment is available. As the population ages, these statistics are expected to worsen.

Previous research and solutions may include: various medications; deep brain stimulation (requiring surgery); wearable vibration device; spoons with stabilization; weighted gloves; or EMG signal filtering. Each of these techniques has one or more deficiencies. Medications may only partially address the problems, and in many cases, lack effectiveness. A person's body may build up a resistance to medications and dosages must be continuously increased. Some medications may carry significant adversarial side effects, e.g., physiological (e.g., kidney), behavioral (e.g., aggression), etc. Surgery to implant deep brain stimulation is extremely costly, and requires invasive surgery fraught with a high rate of fatal risks and severe side effects. Wearable vibration devices, such as in the Emma Project by Microsoft Corporation, have not yet established efficacy. Further, Emma has no assist mode (e.g., to give strength to the user to augment atrophied muscles), and has no exercise mode. A special spoon or fork having self-stabilization may mitigate a solution that works only for eating utensils. Weighted gloves are passive, and simply add weights in the gloves. This solution does not mitigate the tremors fully, and has a low efficacy for severe tremors and loss of strength. The gloves do not provide analysis, patient monitoring, or exercise capabilities. While early research on filtering EMG signals is being performed to find a way to suppress tremors, its efficacy has not been established. EMG filtering may also cause problems by interfering with the intended motions in attempt to filter tremors. EMG filtering does not monitor user condition, nor supplement for the partial loss of strength.

WO 2017 / 062755 A1 relates to an exoskeleton device that allows patients to move a part of their body that they are unable to move by muscle power due to a paralysis. The device is configured to detect motion of another body part that the patient is able to move, infers what movement he wants to achieve with the impaired body part, and effects the movement using actuators.

US 2017 / 014625 A1 and US 2005 / 171577 A1 relate to devices for stimulating muscles in response to measuring an attempted motion and predicting the full motion.

US 9 314 190 B1 relates to a system for providing treatment recommendations for movement disorders. The system comprises sensors configured to measure a patient's motion and a processor to automatically determine treatment recommendations for a physician, based on the sensor data. Furthermore, a drug delivery system is disclosed that allows releasing a drug into a patient's body.

GB 2 444 393 A relates to a device for suppression of muscle tremors. It comprises sensors to detect the tremors and piezoelectric actuators to generate a motion that counteracts the tremor.

### BRIEF DESCRIPTION OF THE DRAWINGS

In the drawings, which are not necessarily drawn to scale, like numerals may describe similar components in different views. Like numerals having different letter suffixes may represent different instances of similar components. Some embodiments are illustrated by way of example, and not limitation, in the figures of the accompanying drawings in which:
FIG. 1 is an illustration of problems caused by neuro-muscular ailments and high-level solutions and applications, according to an embodiment;
FIG. 2 illustrates various scenarios where an assistive device may be used, according to an embodiment;
FIG. 3 is a diagram illustrating a high-level method for a motion modulation apparatus in an assistance system, according to an embodiment;
FIG. 4 illustrates a dynamic continuum of an assistive device system, according to an embodiment;
FIG. 5 is a block diagram illustrating various components of the assistive device system, according to an embodiment;
FIG. 6 is a block diagram illustrating an alternate inference engine of an intended motion inferencer (IMI), according to an embodiment;
FIGS. 7A-7C comprise a block diagram further illustrating various components of the assistance system as shown in FIGS. 5-6, according to an embodiment;
FIGS. 8A-8D comprise a flow diagram illustrating a method for assisting a user in mitigating effects of neuro-muscular ailments in various operational modes, according to an embodiment; and
FIG. 9 is a block diagram illustrating an example of a machine upon which one or more embodiments may be implemented.

### DETAILED DESCRIPTION

The invention is defined by the claims.

In the following description, for purposes of explanation, various details are set forth in order to provide a thorough understanding of some example embodiments. It will be apparent, however, to one skilled in the art that the present subject matter may be practiced without these specific details, or with slight alterations.

An embodiment of the present subject matter is a system and method relating to improving functionality of those suffering from degenerating motor control, and extend the capacity of their independent living. Additionally, the present subject matter includes a physical therapy modality that will help to reduce muscle deterioration, in an embodiment.

The embodiments described herein provide a number of benefits to users at several stages of degenerative disease progressions, such as inferring a user's intended motion to more accurately perform that motion, mitigate tremors, appropriately supplement loss of muscle strength, or provide therapy exercises for users. Further, a user's progression may be monitored for self-customization or for reporting to medical professionals, pharmaceutical institutions, or research institutions among others. It should be noted that embodiments are not limited to human applications, but may also be implemented for animals such as dogs, horses, cats, etc.

Reference in the specification to "one embodiment" or "an embodiment" means that a particular feature, structure, or characteristic described in connection with the embodiment is included in at least one embodiment of the present subject matter. Thus, the appearances of the phrase "in one embodiment" or "in an embodiment" appearing in various places throughout the specification are not necessarily all referring to the same embodiment, or to different or mutually exclusive embodiments. Features of various embodiments may be combined in other embodiments.

For purposes of explanation, specific configurations and details are set forth in order to provide a thorough understanding of the present subject matter. However, it will be apparent to one of ordinary skill in the art that embodiments of the subject matter described may be practiced without the specific details presented herein, or in various combinations, as described herein. Furthermore, well-known features may be omitted or simplified in order not to obscure the described embodiments. Various examples may be given throughout this description. These are merely descriptions of specific embodiments. The scope or meaning of the claims is not limited to the examples given.

Embodiments described herein differ from such technologies as virtual arms, low pass filters, or simple exoskeletons. An embodiment may include elements such as contextual awareness, intention inference, awareness of the user's physical state, selective amplification (e.g., corresponding to the amount of human strength reduction of the select muscles that would need to be involved for a given task), or continuous monitoring of a user's strength or flexibility.

An exoskeleton solution in and of itself (assuming this solution exists) might promote muscle atrophy. An embodiment helps to delay the expected muscle deterioration by limiting assistance to that needed to complete a task given a current state of muscle (e.g., the degree to which the muscle is weakened), and by promoting exercise for weakened muscles. The supplemental force is targeted (e.g., it corresponds to the weakened muscles) and modulated (e.g., the amount of force only replaces, or does less than replace, the muscle strength that has already been lost). Although the descriptions below often focus on solutions for hands, it will be understood that the techniques and methods described herein may be used for any extremity for which tremor suppression or strength augmentation is beneficial. For example, an embodiment may be integrated with leg braces to increase stability, enhance "up and go" movements, or improve posture. In another example, an embodiment may provide balance exercises and assess the state of postural dominant Parkinson's disease. In a further example, an embodiment may include a cervical or neck device to keep one's head erect if neck muscles are weak, etc.

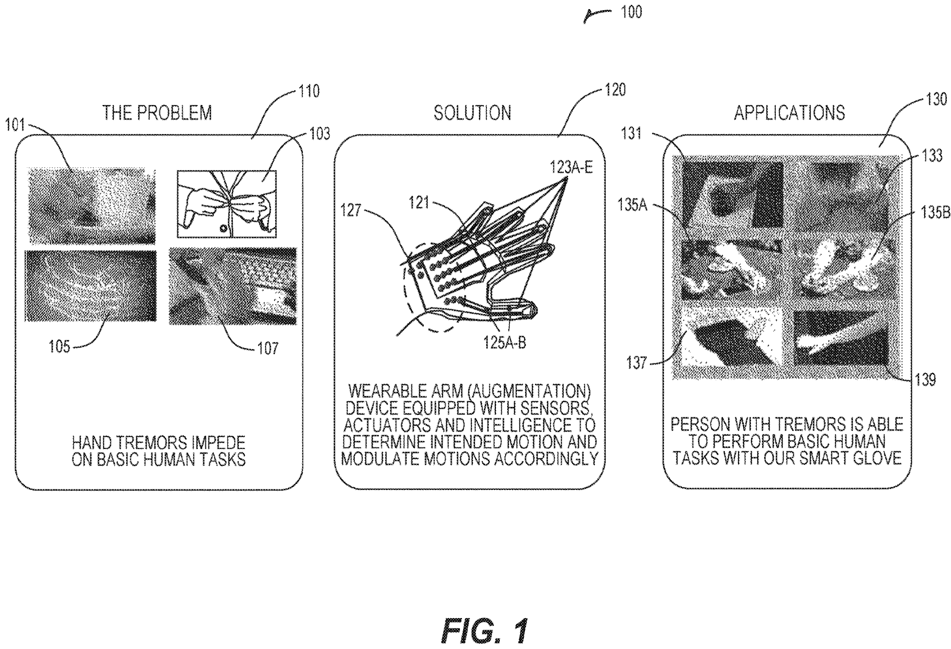

FIG. 1 is an illustration 100 of problems 110 caused by neuro-muscular ailments in the hands or arms of a patient, along with strength level solutions 120-e.g., modifying a user's strength-to these problems that are applicable to several applications 130, according to an embodiment. When an individual experiences tremors in the hands, everyday tasks become more difficult. For instance, holding a coffee cup 101 without spilling the scalding coffee may be virtually impossible when one has severe tremors. Similarly, buttoning a shirt 103, performing a handshake 105, or typing on a keyboard 107 may become onerous tasks.

In an embodiment, a solution 120 to hand tremors includes a glove 121. The glove 121 may include connectors 123A-E, such as cables, ribbon cables, hydraulic lines, pneumatic lines, shape changing materials (e.g., shape memory alloys, smart polymers, etc.), piezo electric lines, etc., to control individual finger joints from actuators 127 (e.g., motors, pumps, etc.). For instance, the glove thumb may include paired connectors 123A and 123B and corresponding actuators 127 for the two thumb joints 125A-B. The actuators 127 control the connectors 123A-E to moderate how much pressure or force is to be applied to a given joint or in which direction vector. In an example, the actuators 127 may initiate or stimulate muscle contraction or relaxation. The glove 121 may be a portion of a wearable arm augmentation equipped with sensors, actuators 127, and intelligence to determine intended motion, and to modulate the arm, hand, or other extremity to correctly implement the user's intended motion. For example, this may include applying different griping pressure depending on an object being grasped (e.g., more griping pressure is applied when griping a bowling ball than when griping an orange).

Applications 130 of the augmentation device may include a variety of tasks. For instance, a user may be able to pick up a glass 131 without dropping it, or applying so much pressure that it breaks. Dexterity may be improved, such as to allow the user to button a shirt 133. The user may able to pick up a slender object such as a smartphone 135A and then use the smartphone with ease 135B. Various form factors of the glove 121 may be implemented, such as a glove with no fingers 137, or a form-fitting glove that extends from the fingertips up to the elbow 139. Different form fits may be useful depending on the user's disability, e.g., tremors vs. loss of strength, etc.

As described above, the glove 121, or other extremity support (e.g., skeletal muscle augmentation device), may be used to help users achieve intended motions, such as picking up a cup, suppressing tremors, etc. Further, a compute node may be used to interpret sensor information to infer the user's intended motions (e.g., hold a cup of water, button a shirt, perform a handshake, etc.).

The compute node may maintain awareness of the user's historical and current muscular strength and flexibility. Actions that correspond to where the user's strength has decayed may be selectively amplified by sending signals to the actuators controlling the device. The system, controlled by the compute node, may also provide an exercise mode for the user to a) perform effective exercise routines, or b) register changes in the user's strength, flexibility, or range of motion. In inferring user intended motion, the compute node may maintain situational and historical context awareness.

In addition to assisting the user with daily tasks and promoting focused exercise to avoid further muscular decay, historical information collected by the device (e.g., in the compute node) may be valuable in ongoing patient care. For instance, feedback data may be of great value to medical professionals (e.g., for disease progression, drug titration, etc.); caregivers (e.g., for creating or monitoring therapy regimens); or the pharmaceutical industry (e.g., for trials, research, etc.). The feedback data may be processed locally (e.g., by the device) or remotely (e.g., raw data from the device is delivered to a cloud component) to produce, for example, feature extraction, historical data, or longitudinal analysis, which may be consumed by a variety of sources (e.g., medical professionals, researchers, etc.).

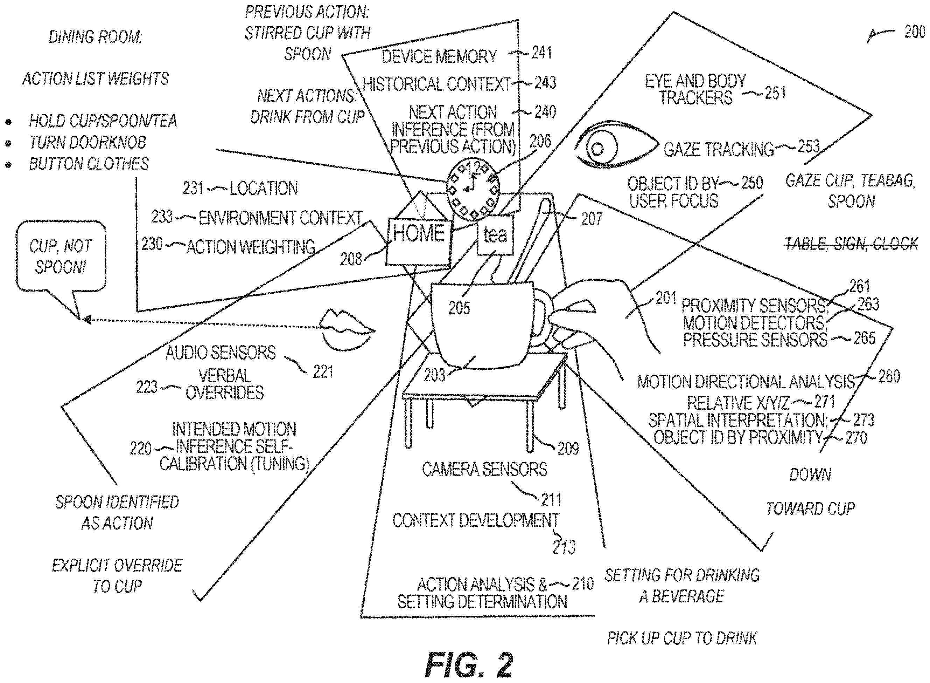

FIG. 2 illustrates various scenarios where an assistive device may be used, according to an embodiment. It should be noted that for illustrative purposes, assistance for a hand or hand-arm combination is described. However, an assistive device may be used for various extremities, core body, or cervical support. Assistance may be provided in a home, office, or public environment, based on available training and sensor information. An environment 200 (e.g., a home environment) is described with various objects with which the user may interact. On the center of the illustration are objects that a user may see in the environment 200. In this scenario a coffee cup 203 rests on a table 209. In the coffee cup 203 may be seen a teabag 205 and a spoon 207. An assistance system may infer or predict the user's intentions toward the objects in the environment 200 based on factors as described more fully below.

In an embodiment, the environment 200 may include camera sensors 211, which may assist in context development 213 for the scene. Action analysis and setting/scene determination 210 may depend on the context. For instance, the visual aspects of the environment 200 as captured by camera sensors 211, and context development 213 based on objects or situation, may assist in the analysis of the scene. In an example, the cup 203 (object) may indicate a setting for drinking a beverage, and a likely action is to pick up the cup 203.

In an embodiment, the environment 200 may include audio sensors 221. Audio sensors 221 may detect auditory cues-such as environment sounds (e.g., breaking glass), verbal cues or overrides 223 (e.g., an exclamation to "stop!," "whoa," etc.), or non-verbal utterances (e.g., a gasp, a moan, a yelp, etc.)-as discussed below. Intended motion inference 220 may use the audio cues for self-calibration (e.g., tuning) 220. For example, the user may perceive that the assistive device is attempting to help move the user's hand 201 toward a spoon 207, but the user's actual intention is to grab the cup 203. In this case, for example, the user may override the device by speaking "cup, not spoon," or "I want the cup." Overrides may be identified-e.g., via training based on the contextual aspects of the scene-by a self-calibration and tuning component.

Location 231 and environmental context 233 may be used to assist with action weighting 230. For instance, it may be more likely that a user plans to drink the coffee (e.g., grasp the cup 203 and bring it to the lips) rather than perform an action related to opening a door. If the user is in a kitchen or dining room, some actions with the coffee cup 203 may be more likely than if at a retail store or in the bathroom. For instance, it may be more likely that the user intends to put an empty coffee cup 203 in the dishwasher when in the kitchen than if at a restaurant. In an example, a list of possible actions may be defined, such as hold cup/spoon/teabag, turn doorknob, or button clothes. A weight may be assigned to each action based on the perceived situational and operational contexts. Weights may be initially assigned manually, or may be assigned based on training criteria, and machine learning. For some contexts, the weight assigned to an action may be 0 or 1 (e.g., for a scale of percentages 0 to 1). Thus, different levels of strength may be applied to grabbing objects; e.g., a stronger grip is used on a ceramic coffee mug and a lighter grip for a foam cup

Device memory 241 with information on previous actions may be used with historical context 243 by a next action inference engine 240 to predict next actions from previous actions related to similar contexts, as discussed above. For instance, if the previous action was "stirred cup with spoon," then it may be inferred that the next action is "drink from cup." It will be understood that when the assistance system is in a passive and reactive mode, predictive inference may be skipped in favor of simple tremor mitigation.

The environment 200 (e.g., employing an assistance system) may include eye and body trackers 251. The system may include a gaze tracking device 253. An object of interest may be identified 250 by user focus as determined by the eye/body tracker 251 or gaze tracking device 253. For instance, when the user's gaze is upon the cup/spoon/teabag (203/207/205) object combination, the inference engine 240 may assign lower weights to actions involving the table 209, home sign 208, or clock 206.

The environment 200 may also include proximity sensors 261, motion detectors 263, or pressure sensors 265. A motion directional analysis engine 260 may use information from sensor(s) 261, 263, or 265 to identify user motion or object motion relative to each other. Relative X,Y,Z coordinate location or movement 271 may be used with spatial interpretation 273 to identify an object by proximity 270. For instance, downward motion may be detected as the user moves their hand 201 down toward the cup 203. When processing motion in space or relations between objects in a three-dimensional space, absolute position may be used (e.g., X, Y, Z coordinates) or relational positioning (e.g., momentum, distance between objects, or orientation such as pitch, yaw, roll, among others) to account for the six degrees of freedom generally available in three-dimensional settings.

It will be understood that not all embodiments will include all sensors as described in environment 200. For instance, when a user is outside of the home, sensors that are not communicatively coupled to the assistive device (e.g., smart glove 121) or on-the-go (OTG) (e.g., smartphone, tablet, wearable device, HMD, etc.) may not provide input to the assistance system. In this case, predictive or real-time inferencing analysis may be limited to available sensor information. In environment 200, for example, user movement toward the cup 203 may predict an action of picking up the cup 203, based on previous actions with these objects in the kitchen environment. The user may intend to grab the spoon 207 instead. For active and predictive help, for instance, the user may need to utter an override if the strength assistance help is too strong to change direction of movement unassisted. In the case of passive and reactive help to mitigate tremors, the user may be able to easily reach for the cup 203 without an explicit override. In an example of an environment with a gaze tracker, the gaze of the user toward the teabag 205 may override the historical context weighting, and correctly predict that the user intends to grab the teabag 205 and not the handle of the coffee cup 203. In an example, when the user is in a home or known environment fit with sensors that may be integrated into the assistance system, various cameras and microphones may be fixed in the environment, as well as coupled to mobile devices worn or held by the user (e.g., wearables, HMD, smartphone, assistive device, etc.). When the user is in an environment with limited or no integrated sensors, then operation of the assistive device may be limited, with some analytics and inference engines omitted or reduced. It will be understood by those of skill in the art, upon review of the present disclosure, that specific functions may be optional or omitted depending on the available sensor and historical data.

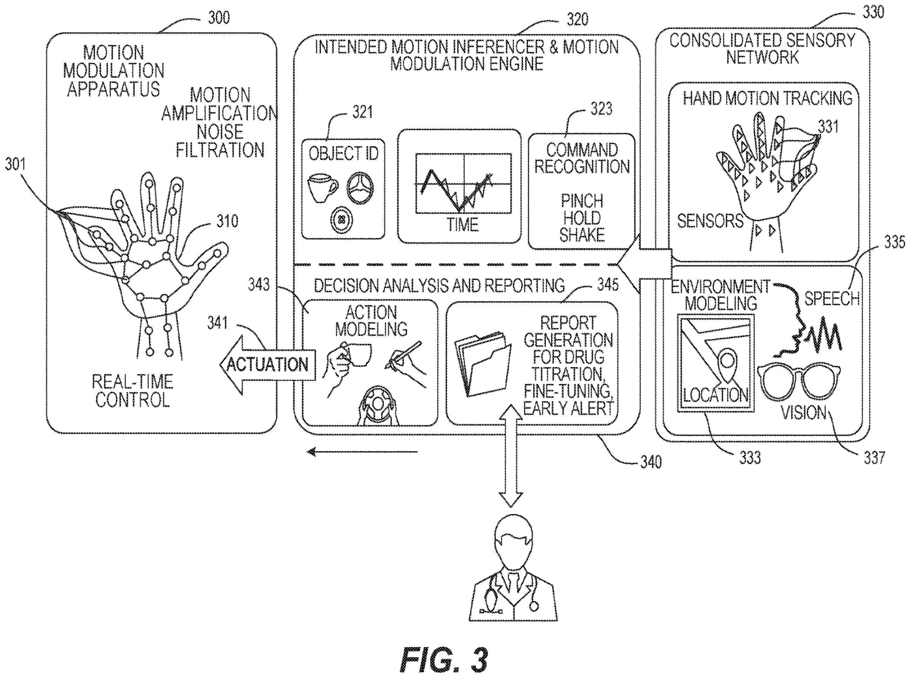

FIG. 3 is a diagram illustrating a high-level method for a motion modulation apparatus 300 in an assistance system, according to an embodiment. Motion modulation apparatus 300 includes a smart glove 310 (e.g., assistive device) having several actuators 301. Actuators 301 are indicated by a small circle on smart glove 310. It should be noted that not all actuators 301 are identified so as not to complicate the figure. The actuators 301 may correspond to pressure or nerve points in a user's extremity (e.g., appendage, hand, etc.). When signals are sent to the actuators 301, current may be applied to the user's extremity to promote or suppress movement in muscles or joints. The smart glove 310 may also include strong and flexible webbing to assist the user with additional strength. The webbing may accept signals to tighten, loosen, or stiffen into a desired shape or juxtaposition, based on the intended action.

In an embodiment, a consolidated sensory network 330 may provide an intended motion inferencer and motion modulation engine 320 with data from a variety of sensors on the assistive device and in the environment. Hand motion tracking 331 using sensors on the assistive device, and environmental modeling using data from location 333, speech 335, and vision 337 sensors, may be used to provide the intended motion inferencer and motion modulation engine 320 with environmental information from the sensors.

In an embodiment, the intended motion inferencer and motion modulation engine 320 may detect motion, and identify objects 321 and the user's intended actions 323 with respect to the objects. As discussed below, various trained machine learning models maybe used to make inferences about the user's intentions based on historical, situational, and operational contexts in a decision analysis and reporting engine 340. The decision analysis and reporting engine 340 may assist real-time control 307 for the motion modulation engine 320 by sending actuation commands 341. In an embodiment, the decision analysis and reporting engine 340 may use action modeling 343 to provide the actuation commands 341. The decision analysis and reporting engine 340 may perform report generation 345 for drug titration, fine-tuning, early alerts, etc. The output of the report generation 345 may be used by a variety of people or institutions to better care for the user. For example, medical professionals may monitor the user's condition, care givers or relatives may be alerted to an incident (e.g., dropped cup), device maintainers may be alerted to device malfunctions, etc.

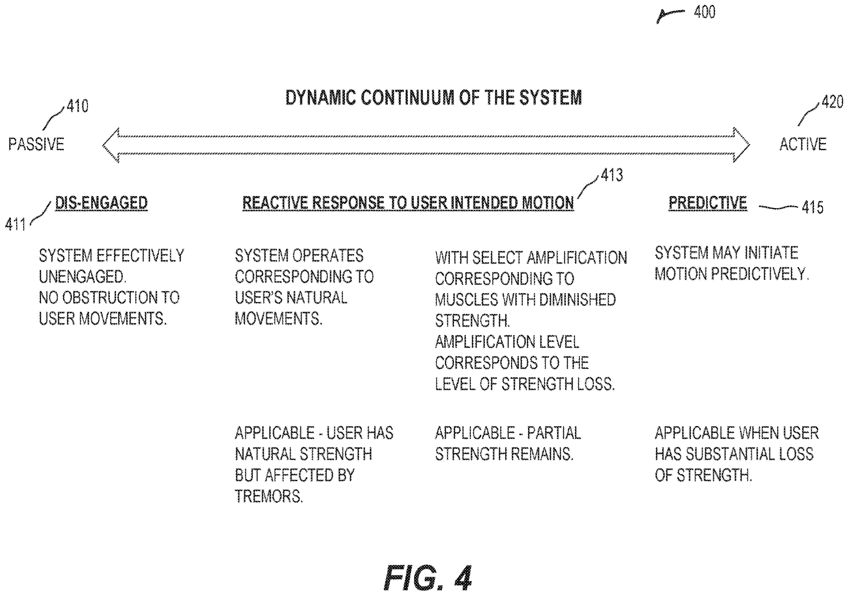

FIG. 4 illustrates a dynamic continuum 400 of the assistive device system, according to an embodiment. The glove may be passive 410 or active 420, or a combination of both. In a completely passive mode 410, the device may be considered to be disengaged 411. When disengaged 411, the device neither assists nor obstructs the user movements. This mode may be used when the user does not need assistance with a specific task, or this mode made be used during an emergency or system malfunction, for example initiated via explicit user command or upon a self-diagnostic indicating a failure.

The device may be in reactive mode 413, where the device is reactive, e.g., in real-time, to user intended motion. The assistive device system may operate corresponding to the user's natural movements while suppressing involuntary tremors. This mode may be used when the user has natural strength but is affected by tremors. Another reactive mode may provide select amplification corresponding to muscles with diminished strength. The amplification level corresponds to the level of strength loss, which may be saved in a data store accessible by the compute node. This mode is applicable when the user has partial strength remaining.

The device may be in a predictive mode 415. In a predictive mode 415 (e.g., autonomous mode), the assistive device may initiate motion predictively (e.g., before the user signals a motion via their muscles) based on situational context, or when the user begins a motion in a situation. This mode is applicable when the user has substantial loss of strength. For instance, if beginning a motion is difficult for a user, e.g., lifting the hand or arm away from the body, the assistive device may predict the action by identifying nearby objects, identifying a time-of-day likely situation, etc. In an example, a user may typically have breakfast at 8AM. At 8:15AM the user faces a coffee cup. Based on the context, the assistive device may trigger muscle reactions to reach for and grab the cup of coffee. The device may be trained to recognize various common scenarios or learn from repeating tasks.

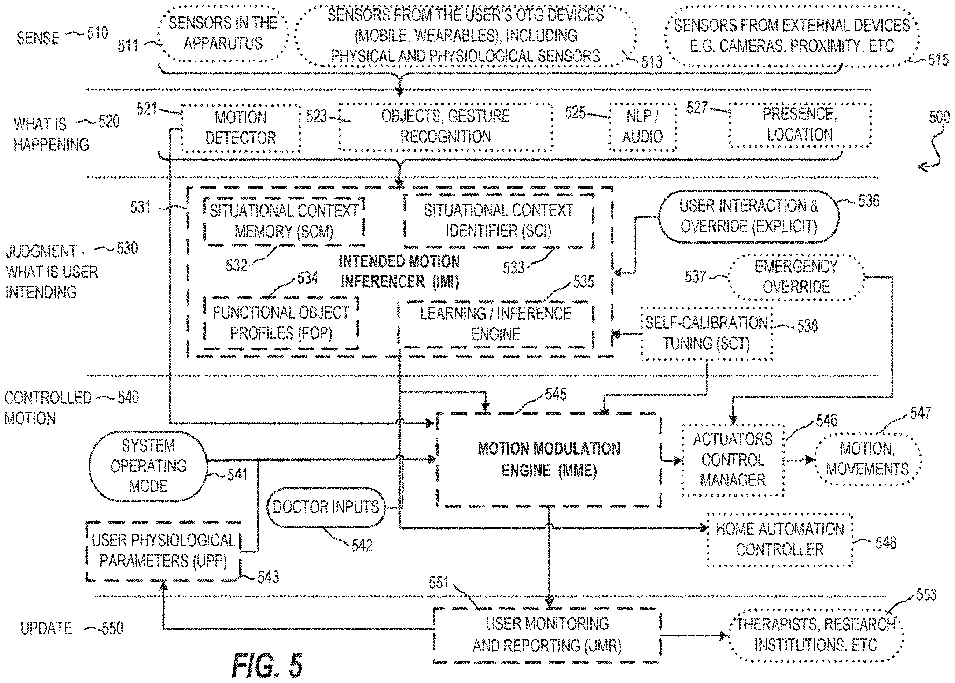

FIG. 5 is a block diagram 500 illustrating various components of the assistive device system, according to an embodiment. For illustrative purposes, components that may be used in existing systems are shown with dotted line borders. Components that are specific to embodiments of the assistive device as described herein are shown with dashed line borders. Integration of these components provides an improved assistive device for users with neuro-muscular problems. The illustration is divided into five functional sections, layers, or modes: sense layer 510; determine what is happening layer 520; judgement (or user intention) layer 530; controlled motion layer 540; and update layer 550.

In an embodiment, the assistive device system comprises a force applicator, such as an exoskeleton-like long glove, that may be implemented with a variety of technologies-such as an electro-mechanical device using electric pumps, motors, or valves to control hydraulic or pneumatic lines, cables or ribbons, etc., or smart polymers, biologic compounds, etc. to apply forces to augment user muscle forces. The force applicator may include multiple sensors (not shown, e.g., motion, pressure, etc.) and actuators (e.g., actuators 127 illustrated in FIG. 1). In one embodiment, the actuators 127 actively suppress motion that is determined to be an unintended tremor. In another embodiment, the glove 121 remains rigid (e.g., instead of actively counteracting tremors-giving the user a feeling that their hand is fixed in the glove 121. This type of device may be comforting for people with hand tremors. Actuators move the mechanical elements of the glove (e.g., ribbons 123 and joints 125), corresponding to the user's intended muscular movements. Actuators motion corresponds to the user's muscle strength exerted (i.e., no amplification) or actuators may selectively "amplify" motion corresponding to the amount of strength lost in particular muscles.

Referring again to FIG. 5, in an embodiment, sensor data may be cumulatively aggregated to determine the user's real-time motion in space (e.g., motion detector) and from the environment for the purposes of contextual awareness. Sensor data may be aggregated from all relevant and available sensor devices. Sensors may include an array of sensors in a smart glove device 511 (e.g., accelerometers, gyroscopes, thermal sensor, pressure sensors, other physiological and movement sensors); sensors in an OTG device 513 or other wearable devices, smartphones, or mobile devices on the user; or sensors in external devices 515 such as cameras, proximity sensors, etc. Sensors on the OTG device 513 may include physical and physiological sensors (e.g., global positioning system (GPS), accelerometer, gyroscope, proximity sensors, microphone, head mounted device (HMD) camera, heart rate monitor, other physiological sensors, etc.). Sensors from external devices 515 such as cameras, proximity detectors, microphones, etc., may be used to provide situational contexts.

In an embodiment, sensor information from sensors in device 511, OTG devices on the user 513, and sensors in external devices 515 are provided to layer 520 to determine what is happening with respect to the user or what is happening in the environment (e.g., the user's situational context). A motion detector 521 may identify the user's movement, and movement of objects in proximity to the user. An object and gesture recognition component 523 identifies user gestures and objects in the user's proximity. Object recognition may assist in situation context, for instance, in the assistance with picking up and holding a coffee cup with liquid. Object recognition may identify that the user is approaching a staircase and may need assistance grasping the railing, or in the case of a leg assistive device, assistance stepping up or down. A natural language processor (NLP) 525 may identify speech from an audio sensor (e.g., microphone). In an embodiment, the user may provide audible (e.g., verbal or non-verbal) commands, or feedback. An embodiment may identify audible sounds other than speech. For instance, a doorbell may be identified and infer that the user is about to get up to answer the door. Or in another example, a whistling tea kettle may cause an inference that the user is about to go into the kitchen to turn off the stove. A presence location component 527 may identify where the user is. For instance, when the user is home, different situational contexts may be relevant as compared to when the user is at work, or shopping. In another example, a proximity sensor may sense that the user is approaching a door that has a particular kind of locking mechanism. In this example, the approach and locking mechanism are aspects of the situational context.

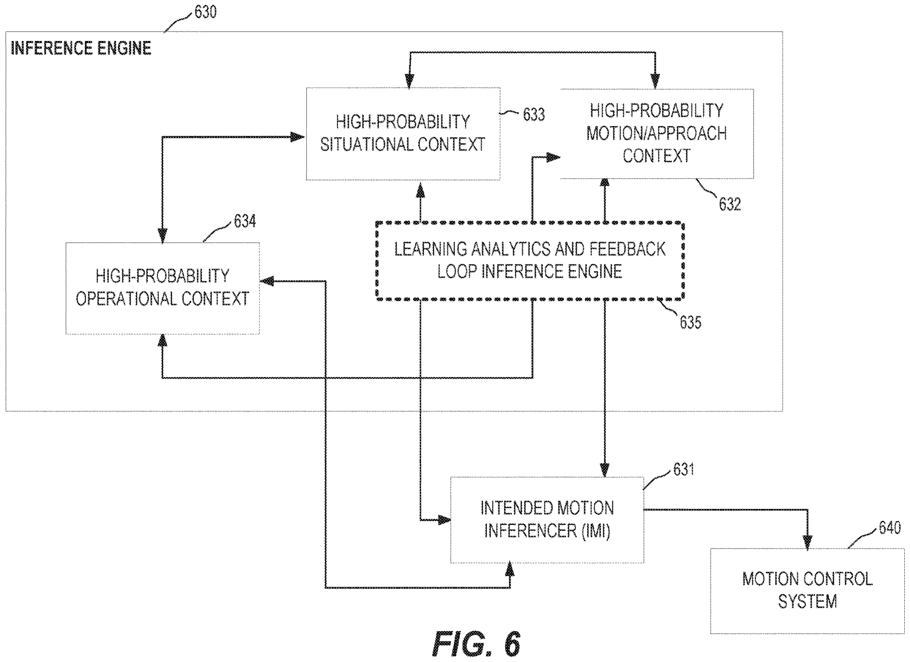

Once the various movement, recognition, language, and location contexts are aggregated and identified in layer 520, the information may be provided to a judgement layer 530. The judgment layer 530 identifies user intention based on movement, objects in the environment, gesture recognition, auditory cues (e.g., verbal or non-verbal utterances, environmental sounds, etc.), or location. In an embodiment, an intended motion inferencer (IMI) 531 may use the information from layer 520 to determine what motion the user intended, which may then be used, for example by a controlled motion layer 540, to provide control or motion modulation information to effectuate the intended motion. In an embodiment, the IMI 531 determines, in real-time, what elements of the user's motion is intended, rather than motion being caused by unintended tremors. The IMI 531 makes determinations by correlating motion with the situational context-for example, by analyzing a motion profile-as well as optionally taking cues from the user, such as with speech and eye gaze, and the environment, for instance for sounds. In an embodiment, in the extreme case of muscle strength loss, IMI 531 may infer the intended motion without relying on the user's muscular motion (as discussed below with reference to FIG. 6).

In an embodiment, the IMI 531 includes a situational context memory (SCM) component 532, a situational context identifier (SCI) 533, functional object profiles (FOP) 534, and a learning/inference engine 535. The IMI 531 may receive input as an explicit user interaction override component 536, for instance, in the event that the user intends to do an unpredictable action, or to correct the IMI 531 when it did not accurately infer the user's intended motion. The IMI 531 may also receive self-calibration and tuning (SCT) information 538 to improve on the inferences made for intended motion.

In an embodiment, SCI 533 determines what is happening with the user and the environment based on the real-time sensor data as well as cues from the prior situational contexts (e.g., retrieved from the SCM 532). SCI 533 provides identification of the situation correlated with historical patterns, location, time and situational context, as well as real-time sensor data. A high-probability outcome is calculated through correlation, inference and standard deviation off of norm. SCI 533 may receive input elements including: environmental context, situational context, and real-time sensor data. The environmental context provides information about the prior location and time indexes, in order to identify any daily patterns and associated locations and time-based events. The situational context associates the prior events, such as correlating information between the environment, location, and the associated things that occur at that location. Real-time sensor data provides additional accuracy and detail for context. SCI 533 uses these inputs to determine high-probability context and ensures improvements towards an increasingly accurate model.

In an embodiment, the SCM 532 provides historical context storage. The historical context is the history of events that occur within the environment and situation, and is used to refine the likelihood of what is going to occur given a present context. Thus, the user may historically eat breakfast every day at 8AM. The IMI 531 may integrate the historical context (e.g., what has happened) from the SCM 532 with the present context from the SCI 533 to more accurately infer user intent. For example, using the historical data in the SCM 532 corresponding to breakfast activities, and a user's movement in the hallway from the SCI 533, the IMI 531 may infer that the user is heading toward the kitchen to begin making coffee. In another example, The IMI 531 may infer that the user is about to get dressed when opening the clothes closet or a dresser drawer at 7:30AM. However, if it is 3PM, opening a dresser drawer may indicate that the user is about to put away clean laundry. Various possible situations may be assigned a probability based on time of day, location, movement, etc. The SCI 533 and the SCM 532 may provide high-probability situational context, which, in conjunction with motion data, is a core input to the IMI 531.

In an embodiment, the SCM 532 assists the SCI 533 by defining relations among motions that have occurred to provide a continuum of motion inference. In an example, a user hand reaches in the direction of a mug with a pen lying next to it. Knowing that a short time ago that the user placed a piece of paper on the table helps to infer that the user is likely reaching for the pen. Knowing that it is breakfast time and that the user recently held a fork helps to infer that the user is likely reaching for the mug. The SCM 532 may assist in providing identification of the situation and events through the correlation of motion (e.g., direction, vector, rate of approach, etc.) and the refinement of the objects in order to preemptively determine the likely object to be manipulated within context. The SCM 532 data may be used in a variety of ways. For example, a motion inference engine, such as in the IMI 531, may evaluate the object and sequence correlation in order to differentiate the response based on the object properties and sequence of events for manipulation. In another example, an approach sequence predictor may provide the specificity and procedure to narrow down the selection of which object is about to be manipulated based on the object that is most likely implicated in the upcoming action.

A FOP 534 may include a list of specific objects with specific corresponding attributes about how those objects may be controlled (e.g., operated). When available, the assistance system performs a lookup in the list based on such information as the user's location. When available, this profile information assists IMI 531 in determining the user's intended motions. For instance, the assistance system may detect the user location on the second floor at the end of the corridor. The look up in the profile list provides information that this is a door to the bathroom with the opener in a shape of a knob that needs to be turned clockwise one quarter turn to open the door. The FOP 534 provides identification of operational context for the objects in the list based on their function and operational modality. This may include standard objects (e.g., available to the general public), modified objects such as assisted (e.g., where the force, grasp, surface is modified to enhance or assist with targeting), and custom systems which are modified to specifically enhance a single user's limitations. FOP 534 may include object operation elements for standard modality, assisted modality, automatic modality, or custom modality. Here, an automatic modality includes those in which the object operates automatically, relieving the user from exerting a force to operate the object. Examples may include an automatic door opener that may be controlled through a building (e.g., home) automation network. Thus, a detected user proximity or verbal command may open or close the door.

The standard modality element or component may engage with objects for operation and predictive/proactive actions based on how everyday objects are in the situation without any enhancement. The assisted modality element may engage with objects for operation and predictive/proactive actions based on some assistive enhancements to everyday objects that allow simpler manipulation, for instance, enhancements related to the Americans with Disabilities Act (ADA) assistance. The custom modality element may engage with objects for operation and predictive/proactive actions based on custom enhancements that are specific to the user, such as home improvements and automobile/driving enhancements. In an example, a communication component (e.g., a transmitter, transceiver, etc.) may send a signal or command to an object requesting the object to assist the user in the pre-defined, custom manner. For instance, in an example, the request may be to open or close an automatic door. The FOP 534 may provide the high-probability operational context, which is a third core input to the IMI 531.

The learning or inference engine 535 may include a feedback to continuously improve the decision making abilities of the assistance system. For instance, when a user verbally interferes with the predicted intent (e.g., overrides the predicted intent with an actual intent), such as when the IMI 531 misjudges the user's intention, a self-learning adjustment may be triggered. An inference engine within 535, as discussed more below in reference to FIG. 6, may comprise high-probability context elements that provide improved accuracy through learning, analytics, and a feedback loop.

In an embodiment, many of the actions shown in FIG. 5 may be processed on the assistive device so that the user does not need to rely on access to a cloud or Internet connection. Some processes may be performed on a local edge cloud, for instance a local server in the user's home or office, or even on a smartphone or other mobile device. To provide more independence for the user, objects, situations, and actions that are common to the user may have local profiles stored in memory on the assistive device. Training of the assistance system, or user monitoring and reporting, may require access to the Internet or the cloud, however, training, monitoring, or reporting may also occur locally. In an example, training may require use of computationally complex and intensive training models which may be better performed offline on a compute node with sufficient power. Access to the cloud or Internet server may be required to receive doctor inputs, profiles for new objects, etc. Self-calibration inputs such as overrides may be applied immediately, or be saved for a later time when the assistive device is connected to a computationally powerful device to retrain the models. It will be understood that varying combinations of processes or components may reside on the assistive device as compared to the local or remote cloud server, as necessary to provide real-time assistance to the user.

FIG. 6 is a block diagram illustrating an alternate inference engine of an IMI, according to an embodiment. In an embodiment, an inference engine 630 receives the core outputs from SCM 532, SCI 533, and FOP 534, in the form of probabilities, including: high-probability motion/approach context 632, high-probability situational context 633, and high-probability operational context 634, respectively. Probabilities are used because the user's motion is not deterministic. User motion is analog, not digital, and has virtually infinite possibilities. Therefore, possible situational context, operational context, and motion/approach context may be assigned a probability before correlation. These high-probability context predictions are used by learning analytics feedback loop inference engine 635 to receive continuous contextual feedback and train the system by correlating operational context, situational context, and motion/approach context. It will be understood that correlations may use any one of these context types as primary-e.g., as a seed, beginning, or starting point, etc.-in the training. For instance, training may focus on the situation first (e.g., the situational context is the seed) and correlate the situation with historical operations (e.g., operational context) and motion (e.g., motion or approach context). Alternatively, the training may focus on historical motion correlated with situation and movement. In an embodiment, the various contexts may be trained as peer variables. It will be understood that various machine learning models may be used to correlate the three types of context as variable input.

Once trained, machine learning models in the learning analytics and feedback loop inference engine 635 may provide one or more high-probability inferences for situation, movement/motion, and operation to an IMI 631. It will be understood that even though the inference engine 630 is illustrated as a separate block or component in FIG. 6, the inference engine 630 may be implemented as a sub-component of IMI 631, or that various components of inference engine 630 and IMI 631 may be implemented as a single process or as distributed process, or a combination. The IMI 631 uses the high-probability inferences (e.g., for situation, movement/motion, and operation) and provides motion information to the motion control system 640 (e.g., layer 540, or MME 545 illustrated in FIG. 5).

Referring again to FIG. 5, the IMI 531 may provide high-probability information as to what the user's intent (e.g., intended motion) is to the motion modulation engine (MME) 545, while the MME 545 uses this information to perform the motion (e.g., augment the user's own muscle inputs to achieve the intended motion). In an embodiment, the MME 545 activates a force applicator of the assistive device (e.g., electro-mechanical apparatus, smart glove, etc.) to implement the intended motion. The MME 545 receives information about the user motion, for instance from the motion detector 521, and the intended motion from IMI 531. Based on the operating mode or other parameters, such as user strength and limitations, or predictive vs. reactive user assist modes. The MME 545 determines which motions to activate, via the actuators (e.g., actuators 127), or through other signaling-such as a radio signal to operate an automatic door or light switch. The MME 545 may determine which actions will utilize the user's innate or current strength, and which actions require amplification or assistance in strength, and for which actuators, to complete the intended motion. Each actuator may provide muscle assistance or tremor suppression to a single muscle or group of muscles. Therefore, if the user has a loss of strength in only some muscles, or varying levels of strength loss, actuators may be activated at different levels of intensity. If the user has tremors in specific muscles, then the actuators corresponding to the muscles prone to tremor may be activated to suppress the tremors. In another embodiment, there is no activation of actuators for intended motion of a user, and the user may complete the motion with innate strength. However, in this case, suppression may be used for unintended motions, e.g., tremors, tics, or other motion disorders.

In an embodiment, the MME 545 helps to implement the three previously described operating modes: system initialization, standard user operating mode, and exercise mode. The MME 545 may operate differently based on receiving a system operating mode flag or identifier 541. The system initialization mode may be performed by a family member, nurse, therapist or other individual to mimic the user's daily routines while operating/wearing the assistive device. Mimicking of the daily routines may be performed to train the assistance system and provide a baseline for correlation of situational, movement, and operational contexts. This training may also populate the object profiles in FOP 534. The standard user operating mode is the mode for user assistance. The MME 545 may include machine learning to learn and fine-tune its operation. Runtime learning may enhance the initial baseline when the assistive device is in operation by the user.

In an embodiment, the assistance system includes an exercise mode. This mode provides an exercise regimen for the user (e.g., stretching and strengthening). In this mode, the system may first prompt the user before engaging in a resistance training course. The MME 545 may locally adjust how much resistance or stretching to use depending on the strength remaining in particular muscle(s), as well as log user progress to generate reports for the user and health providers. Exercise regimens and recommendations may include doctor inputs 542. Log data may also be used to calibrate the operation of the device in standard user operating mode.

MME 545 may also utilize additional attributes, such as user physiological parameters (UPP) 543, and doctor inputs 542. The UPP 543 may include a database of the user's current health state, e.g., strength and flexibility, corresponding to particular muscles and joints. The UPP 543 may be continuously updated by the system based on the processions of the exercise mode, or received from external sources, e.g., doctors, physical therapist. Doctor inputs 542 may provide information regarding which muscle(s) to exercise in particular, and which exercise(s) are recommended, including frequency and duration or number of repetitions. In an embodiment, the UPP 543 may also include specific directives to more aggressively suppress tremors (e.g., because it is comforting to the user), or conversely to not suppress tremors (e.g., because, in some conditions, it is uncomfortable to the user).

The controlled motion layer 540 may also include an actuators control manager (ACM) 546, a home automation controller 548, and a motion or movement initiator 547. ACM 546 may drive the network of actuators in the assistive device (e.g., smart glove) to execute a particular motion based on the direction/instructions from the MME 545. The motion or movement initiator 547 may send signals or instructions to the individual actuators to effect the movement. A home automation controller 548 may communicate with a home automation type of network and devices. For instance, the IMI 531 may determine that the user is intending to open the door to the bathroom. As an alternative to requiring the user to open the door manually, the IMI 531 may send a command to the home automation controller 548 initiating a command to open an automatic door via the home automation network, when available with a digital door opener.

In an embodiment, the assistance system may include an emergency override 537 to indicate that the assistance system immediately stops and removes any suppression/amplification/interference to the user motion. This override 537 may be based on a particular voice command (keyword, exclamation, moaning, gasping, yelping, etc.), gesture, or activation of a particular "stop" button/switch, etc. For instance, when a user feels that device is not cooperating, or is providing an incorrect assistance/suppression, the user may deactivate the assistive device (e.g., send an immediate signal to the actuators control manager 546 to deactivate the actuators). Deactivation may be triggered by a physical or virtual switch or button, or by voice command. In an embodiment, deactivation (e.g., emergency override) may be triggered by someone other than the user, such as a family member, caregiver, emergency response personnel, medical professional, etc.

In an embodiment, the assistance system may include a user interaction override component 536 to provide corrections to the IMI 531 when the user perceives that the device is making incorrect predictions, but an emergency shutdown is not necessary. For instance, the IMI 531 may infer that user is reaching for the cup. The user may speak aloud, "I am trying to pick up the spoon." This command or movement suggestion may be identified with NLP 525 and used by the IMI 531 to correct the action. Situational, operational, and motion contexts for this correction may be input to the learning/inference engine 535 for additional training. It should be noted that other cues may be used to override the IMI 531. For instance, a particular motion such as a sharp jerk of the hand or pulling the hand back and forth, or other gesture, may be pre-defined to indicate to IMI 531 that the inference decision was inaccurate.

In an embodiment, the assistance system may include a self-calibration tuner (SCT) 538. The SCT 538 may include a continuous self-learning platform for IMI 531 and MME 545. In a simple form, for IMI 531, the SCT 538 may adjust the machine learning weights for a model, when a user explicitly overrides the IMI 531 inference. Self-calibration of MME 545 may be performed when the user expressed that a particular motion was either insufficient (e.g., fingers did not enclose the door knob sufficiently to turn the knob), or that the motion created discomfort in the user (e.g., due to excessive force from the actuators).

In an embodiment, the MME 545 may provide historical information to an update layer 550. The update layer 550 may include a user monitoring and reporting (UMR) 551 component. UMR 551 may provide reports or analysis to outside persons or entities (e.g., via the cloud 553), such as therapists, research institutions, etc. UMR 551 may also provide updated information on the user's strength levels and other current operation information to UPP 543 for use with the MME 545. During assistance mode, for instance during the user standard operating mode, and especially during the exercise mode, the assistance system may continuously assesses the user's current muscle strength, flexibility, and motor control. UMR 551 may update the UPP 543 database. UMR 551 may also monitor a range of physical attributes (e.g., strength, tremor intensity, biometrics, and particular activities).

UMR 551 may be enhanced by adding objective assessment per a pre-defined scale such as the Unified Parkinson Disease Rating Scale (UPDRS). In an example, timing tests may be performed to see how many times the patient may touch their index finger to the thumb in a specified interval, or how many times the user may pronate/supinate their hands, as measures of dexterity. A subset of tests that may be easily automated by the assistive device may be run regularly (e.g., weekly or biweekly). These strength and dexterity tests may be performed much more frequently than if performed only at an annual/semi-annual physical, which is currently typical for patients. The results of these tests may be reported up to the cloud 553, for use by doctors, therapists, and other concerned parties. This data may be of great value to doctors, therapists, pharmaceuticals, research institution, etc., to provide better drug titration, physical therapy (e.g., exercises), or to help other similarly situated individuals. In an embodiment, the information is processed and particular feature set(s) provided with appropriate degrees of privacy protection, or encryption.

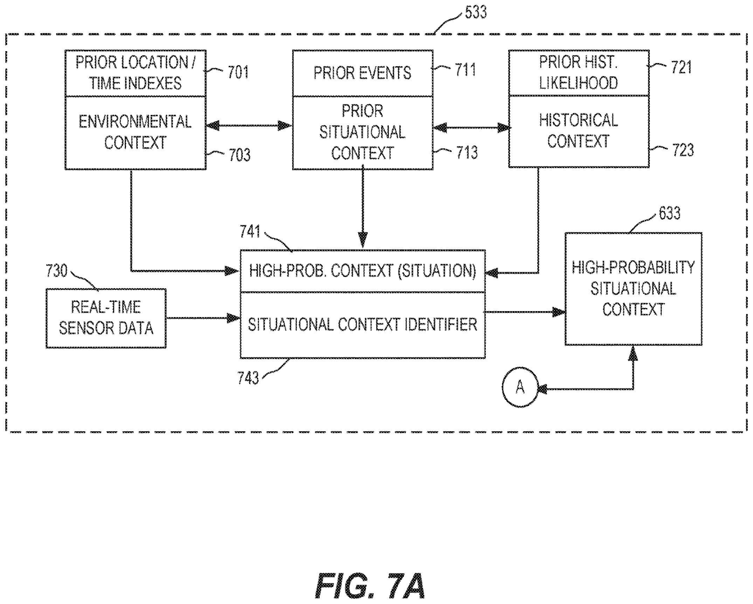

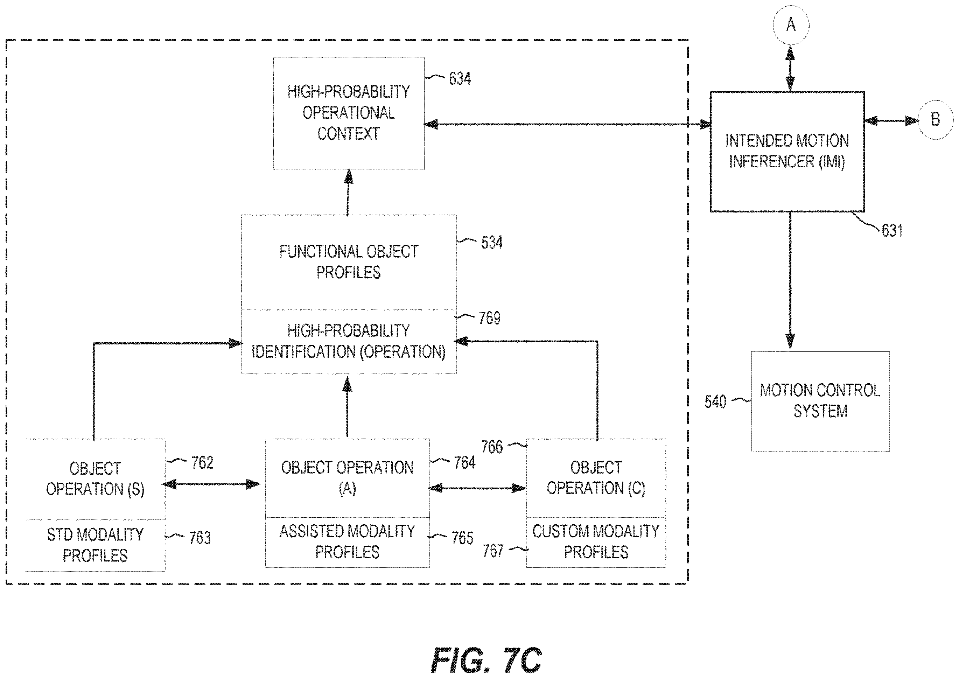

FIGS. 7A-7C comprise a block diagram further illustrating various components of the alternative assistance system as that illustrated in FIGS. 5-6, according to an embodiment. FIG. 7A illustrates a SCI 533 component (e.g., shown in FIG. 5), according to an embodiment. A situational context identifier 533 may use high-probability context (situation) input 741 to identify the high-probability situational context 633, which may be used by the IMI (631, FIG. 7C). The situational context identifier 743 may use several input data 730, 741 and derived inputs such as contextual identifiers 703, 713, and 723 to identify the situational context. The high-probability context (situation) 741 may be derived from environmental context 703, prior situational context 713, and historical context 723. Environmental context 703 may use prior location and time indexes 701 to identify an environment (e.g., home, office, grocery store, restaurant, hallway, kitchen, bathroom, etc.). Prior events 711 may be used to identify prior situational context 713 (e.g., in kitchen may mean eating or drinking requiring use of utensils). Historical context 723 may use prior historical likelihood information 721 (e.g., in kitchen with cup object present means picking up the cup is likely). The situation contextual identifier 743 may use high-probability context (situation) 741 and real-time sensor data 730 to generate the high-probability situational context 633. In an embodiment, a trained machine learning model may be used at one or more points in the determination/calculation for the various contextual identifiers 703, 713, 723, or 741 where the model is specific to the input 701, 711, 721, 730, or 741 data used to derive the contextual identifiers 703, 713, 723, 743, or 633/741.

In an example, the user enters a local coffee shop known to use paper cups. The situational context may indicate a certain pressure to be placed on the cup to hold it safely. Another coffee shop may use ceramic cups which require a different pressure to hold. If the user has frequented both coffee shops, both locations and default cup profiles may be saved as prior situational context as applied to the specific objects. Therefore, when the user enters the shop that uses ceramic cups, the location probability will correlate with the object or operational probabilities to apply the appropriate pressure. It is possible that the shop will use a different kind of cup that day, or that the coffee shop is not known. In those cases, the user might utter "I am using a paper coffee cup" to alert the assistive device to choose the correct object profile, as discussed more below.

In an embodiment, the user may indicate through audible or other means that the user intends to visit the coffee shop that uses the ceramic cups. Historical context may infer a series of actions to travel to the coffee shop, order and drink the coffee. When the user is away from home, only a subset of historical actions and functional object profiles may be available in local storage of the assistive device. For instance, the user may know that network connectivity is unreliable at the coffee shop. This situational context may be stored as a profile for the location or activity in a remote cloud server or local edge cloud server. In an embodiment, various historical actions and functional object profiles or a series of expected actions may be pre-fetched from the cloud based on the intended situational and operation context (e.g., going to the coffee shop). Therefore, the assistive device may operate at a higher level of accuracy in inferring the intended motion and actions when the data is pre-fetched than if it had to rely on the subset of information typically stored locally.

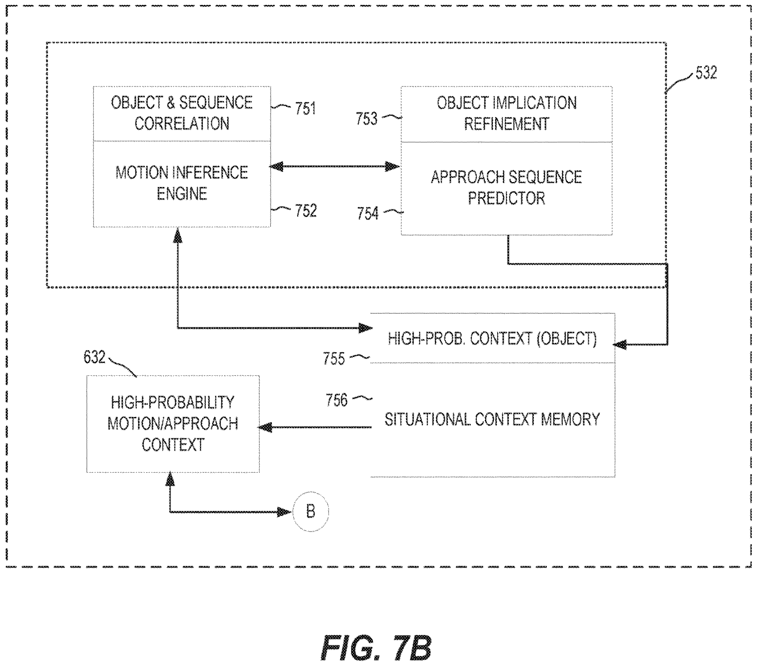

Referring now to FIG. 7B, there is shown a block diagram illustration of the situation and events through the correlation of motion, according to an embodiment. Identification of situation and events may be derived from the correlation of motion (direction, vector, rate of approach) and the refinement of the objects in order to preemptively determine the likely object to be manipulated within context. As discussed above, SCM 532 (FIG. 5) may include a motion inference engine 752 and an approach sequence predictor 754. The motion inference engine 752 evaluates the object and sequence correlation 751 in order to differentiate the response based on the object properties and sequence of events for manipulation. The approach sequence predictor 754 provides the specificity and procedure to narrow down the selection of which object is about to be manipulated based on the object that is most likely implicated in the upcoming action. Object implication refinement 753 is a technique by which refinements to object selection-e.g., which object is about to be used-are made. For example, when approaching several objects, such as a pen, notepad, newspaper, and a cup on a table, it may be difficult to determine which of the several objects the user intends to interact. However, given a pattern of user behavior to grab the cup after putting down the pen, the object selection may be refined to select the cup with a greater probability than the newspaper, for example, if the pen were recently put down. The SCM 532 may provide input to the high-probability context (object) 755. The high-probability object context 755 may be used to determine the situational context memory 756 having both motion inference and approach sequence information regarding the object (e.g., operational context). Situational context memory 756 may provide the high-probability motion/approach context 632 to the IMI 631 (FIG. 7C).

Referring now to FIG. 7C, there is shown a block diagram illustrating identification of the operational context for the objects 634 (FIG. 6), according to an embodiment. The high-probability operational context 634 may be identified based on the object function and operational modality. Objects may include standard objects (e.g., available to the general public, like paper or ceramic coffee cups), modified objects such as assisted objects (e.g., where the force, grasp, surface is modified to enhance or assist with targeting), and custom modality objects which are modified to specifically enhance a single user's limitations. High-probability identification (operation) 769 may be derived from object operation (Standard) 762, object operation (Assisted) 764, or object operation (Custom) 766. Object operations (Standard) 762 may use standard modality profiles 763 to identify a user's likely operation for the standard object. Object operations (Assisted) 764 may use assisted modality profiles 765 to identify a user's likely operation for the assisted object. Object operations (Custom) 766 may use custom modality profiles 767 to identify a user's likely operation for the custom object. For instance, the object may be identified as a coffee cup without a handle, which may assign a higher probability to paper cup or expanded polystyrene foam cup than to a ceramic cup.

FOP 534 operation (e.g., shown in FIG. 5) provides identification of operational context for the objects based on their function and operational modality as determined for the high-probability identification (operation) 769. The functional object profiles 534 may provide the input to the IMI 631 to determine the high-probability operational context 634 (FIG. 6). The functional object profiles 534 generally store information related to objects and how they are operated. For example, a functional object profile for a kitchen microwave may include: where it is located (e.g., what room, how high off of the floor, over a counter, etc.); where is the handle (e.g., on the left side, the top, etc.); where is the start button, what particular signals (e.g., sounds, lights, etc.) it may emit, among other things. In another example, the profile for a door may include the type of opening mechanism the door has, including details such as where the knob is placed, how it turns (e.g., clockwise or counter-clockwise), or whether the door has an electromechanical door opening capability. As discussed above, the IMI 631 provides intended movement information to the motion control layer 540, which in turn controls a force applicator to effectuate the intended motion.

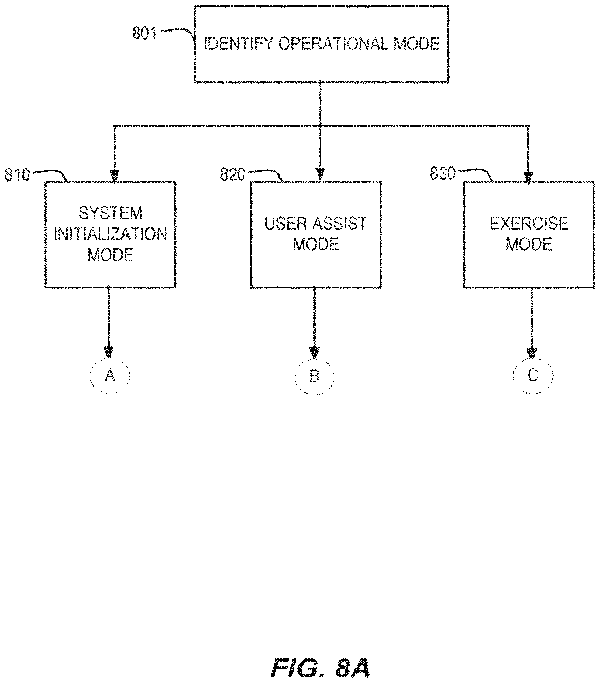

FIGS. 8A-C comprise a flow diagram illustrating a method for assisting a user in mitigating effects of neuro-muscular ailments in various operational modes, according to an embodiment. An assistance system identifies the operational mode in block 801. The assistive device may operate in system initialization or setup mode 810, user assist mode 820, or exercise mode 830.

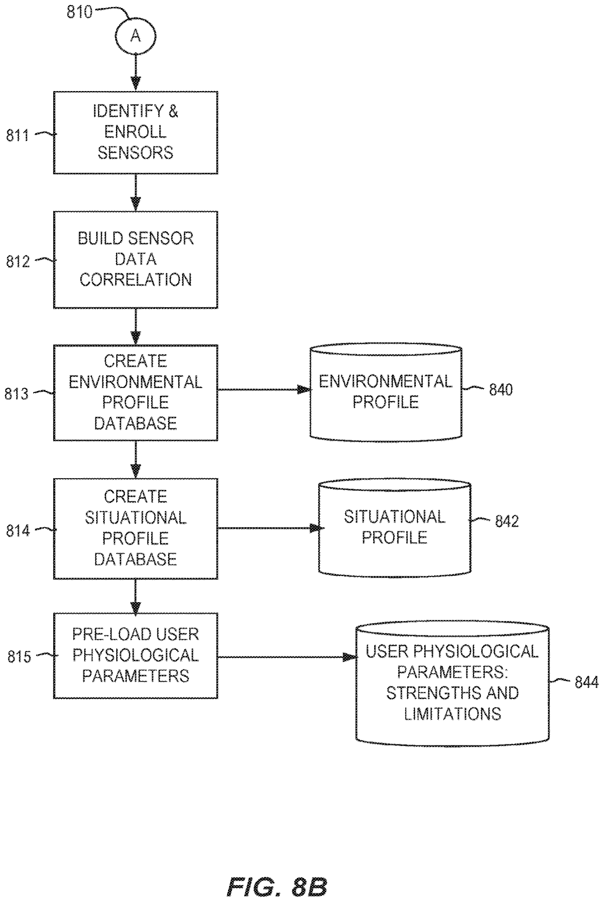

Referring now to FIG. 8B, a flow diagram is illustrated for a method of system initialization 810, according to an embodiment. Sensors available for input may be identified and enrolled in block 811. A method for auto-discovery may be used to identify sensors in range. Alternatively, a user may manually locate and enroll sensors. Sensors may include those on the assistive device, other OTG or wearable devices, and environmental devices. A data correlation map for the enrolled sensors may be built in block 812. Sensor data correlation may establish relationships between sensors-such as where are they with respect to each other, where are they with respect to the environment (e.g., location, elevation, etc.), how their data may be correlated (e.g., in time, in overlapping field of views, etc. based on which intention inference is made. An environmental profile database 840 may be created in block 813. A situational profile database 842 may be created in block 814, and used to capture a situational context and, for example, inform the environmental profile database 840. The environmental profile and situational profile databases 840, 842 may collect some sensor data from the environment, as well as use training data to populate the profiles. In an example, a nurse or family member may walk through the home environment. A camera, such as coupled to a head mounted display (HMD) (e.g., glasses, visor, helmet, etc.) or mounted on a wall, may capture environmental information, e.g., located in hallway, bathroom, kitchen, family room, etc. Situational context may be entered or trained using common activities of the user in those environments.

Initial physiological parameters of the user may be pre-loaded into a user physiological parameter (UPP) database 844, in block 815. User strength and limitation information may be assumed, received from a health care professional, or derived from testing, and entered as a baseline. It will be understood that the UPP database 844 may be populated before or after the environmental profile data base 840 and situational profile database 842.

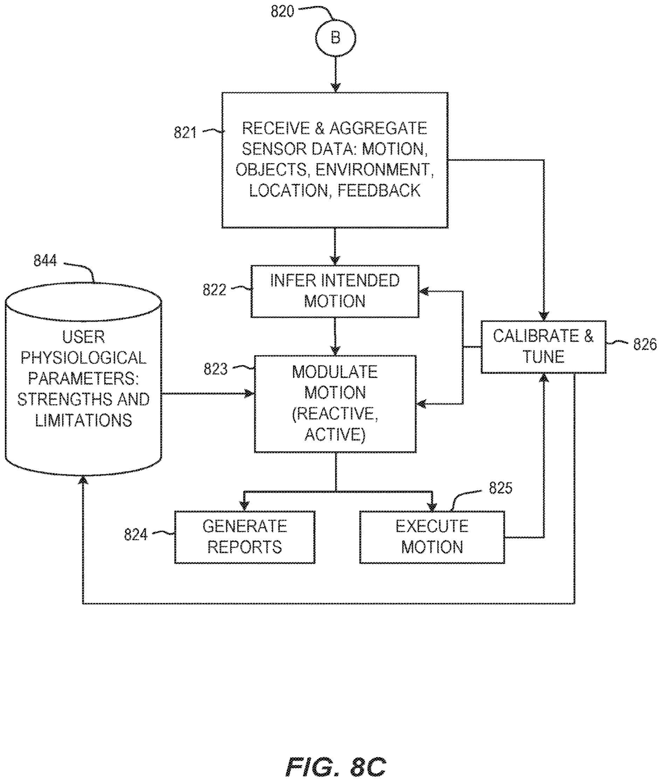

Referring now to FIG. 8C, a flow diagram is illustrated for a method of user assist mode 820, according to an embodiment. It will be understood that user assist mode 820 may have various levels of passive, reactive, or active assistance across the dynamic continuum, as illustrated in FIG. 4. When the assistive device is in user assist mode 820, the assistance system receives sensor data for a variety of contexts or actions, including but not limited to, movement, object identification, location, environment, and user feedback, in block 821. The sensor data may be aggregated to correlate information from various sensors. Historical context may also be correlated with the sensor data to derive a real-time context. As discussed above, various machine learning models may be used to assist in the correlation of data. When in predictive mode, intended motions may be inferred in block 822. In reactive mode, intended and unintended motion may be inferred, as well, to inhibit the unintended motion. In an example, a user may have very little strength to provide a predictive motion from movement. In this case, motion may be predicted based on context. Motion may be modulated in block 823 to alleviate tremors, unintended motion, or provide assistive strength for the intended motion. Motion modulation may be reactive instead of being predictive, modulating and executing an action. The motion modulation may be based on the user's strength and limitation parameters 844. Motion modulation may include sending signals 825 to actuators in an assistive device such as a smart glove, flexible leg brace, cervical support, etc. It will be understood that motion modulation may be active (e.g., strength assistive) or reactive (e.g., tremor mitigation), or both, as identified by the operational mode and user physiological parameters (e.g., using the UPP database 844). User monitoring and reporting, as discussed above, may be performed in block 824. Calibration and tuning of the assistance may be performed in block 826, as discussed above.

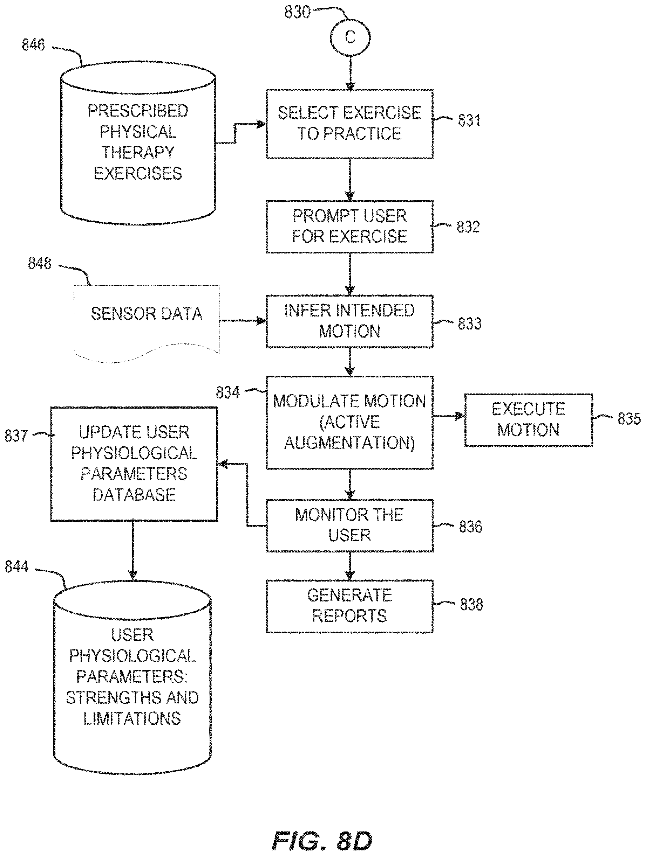

Referring now to FIG. 8D, there is shown a flow diagram illustrating a method for exercise mode 830 of the assistive device, not forming part of the present invention. The user, or the system, may select an exercise to practice in block 831. When the system selects the exercise mode 830, the system may prompt the user to start or engage in the activity. In an embodiment, the exercise may be retrieved from a list of prescribed physical therapy exercises 846. The exercises may be suggested to the user on a periodic basis, or require manual selection. Various automatic reminder modes may be pre-selected by the user. Once selected, or suggested, the user may be prompted to begin the exercise in block 832. Sensor data 848 identifying the user motion may be collected and used to infer intended motion in block 833. Motion may be modulated, as necessary, in block 834, and signals sent to actuators to assist intended motion or to inhibit unintended motion in block 835. The user is monitored by sensors in the assistive device, in block 836. Strength levels may be inferred based on movement, pressure, and other sensors in the device. Changes in user strength or limitations or other physiological parameters may be updated in the UPP database 844, in block 837. Reports on status of exercise regime and updated parameters may be generated, in block 838. The reports may be stored for later review, or forwarded to the user or health care provider as desired.

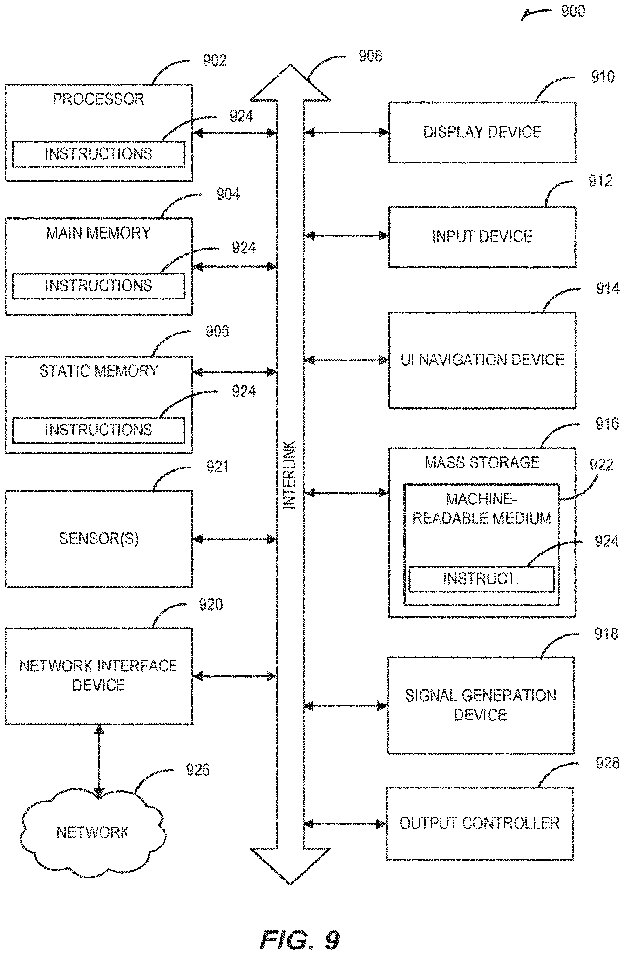

FIG. 9 illustrates a block diagram of an example machine 900 upon which any one or more of the techniques (e.g., methodologies) discussed herein may perform. In an embodiment, the assistive device (e.g., smart glove or leg brace, etc.) communicates with the machine 900 (e.g., a server machine) which may be used to execute the trained models and provide the motion controls based on inferred intended movement, according to the contextual data. The machine 900 may be a local or remote computer, or processing node in an OTG device such as a smartphone, tablet, or wearable device. The machine 900 may operate as a standalone device or may be connected (e.g., networked) to other machines. In an embodiment, the machine may be directly coupled or be integrated with the assistive device. It will be understood that when the processor 902 is coupled directly to the assistive device, that some components of machine 900 may be omitted to provide a lightweight and flexible device (e.g., display device, UI navigation device, etc.). In a networked deployment, the machine 900 may operate in the capacity of a server machine, a client machine, or both in server-client network environments. In an example, the machine 900 may act as a peer machine in peer-to-peer (P2P) (or other distributed) network environment. The machine 900 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. Circuitry is a collection of circuits implemented in tangible entities that include hardware (e.g., simple circuits, gates, logic, etc.). Circuitry membership may be flexible over time and underlying hardware variability. Circuitries include members that may, alone or in combination, perform specified operations when operating. In an example, hardware of the circuitry may be immutably designed to carry out a specific operation (e.g., hardwired). In an example, the hardware of the circuitry may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuitry in hardware via the variable connections to carry out portions of the specific operation when in operation. Accordingly, the computer readable medium is communicatively coupled to the other components of the circuitry when the device is operating. In an example, any of the physical components may be used in more than one member of more than one circuitry. For example, under operation, execution units may be used in a first circuit of a first circuitry at one point in time and reused by a second circuit in the first circuitry, or by a third circuit in a second circuitry at a different time.

Machine (e.g., computer system) 900 may include a hardware processor 902 (e.g., a central processing unit (CPU), a graphics processing unit (GPU), a hardware processor core, or any combination thereof), a main memory 904 and a static memory 906, some or all of which may communicate with each other via an interlink (e.g., bus) 908. The machine 900 may further include a display unit 910, an alphanumeric input device 912 (e.g., a keyboard), and a user interface (UI) navigation device 914 (e.g., a mouse). In an example, the display unit 910, input device 912 and UI navigation device 914 may be a touch screen display. The machine 900 may additionally include a storage device (e.g., drive unit) 916, a signal generation device 918 (e.g., a speaker), a network interface device 920, and one or more sensors 921, such as a global positioning system (GPS) sensor, compass, accelerometer, or other sensor. In an example, sensors 921 may include wearable, assistive device-based and environmental sensors, as described above. The machine 900 may include an output controller 928, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 916 may include a machine readable medium 922 on which is stored one or more sets of data structures or instructions 924 (e.g., software) embodying or utilized by any one or more of the techniques or functions described herein. The instructions 924 may also reside, completely or at least partially, within the main memory 904, within static memory 906, or within the hardware processor 902 during execution thereof by the machine 900. In an example, one or any combination of the hardware processor 902, the main memory 904, the static memory 906, or the storage device 916 may constitute machine readable media.

While the machine readable medium 922 is illustrated as a single medium, the term "machine readable medium" may include a single medium or multiple media (e.g., a centralized or distributed database, or associated caches and servers) configured to store the one or more instructions 924.

The term "machine readable medium" may include any medium that is capable of storing, encoding, or carrying instructions for execution by the machine 900 and that cause the machine 900 to perform any one or more of the techniques of the present disclosure, or that is capable of storing, encoding or carrying data structures used by or associated with such instructions. Non-limiting machine readable medium examples may include solid-state memories, and optical and magnetic media. In an example, a massed machine readable medium comprises a machine readable medium with a plurality of particles having invariant (e.g., rest) mass. Accordingly, massed machine-readable media are not transitory propagating signals. Specific examples of massed machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 924 may further be transmitted or received over a communications network 926 using a transmission medium via the network interface device 920 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 920 may include one or more physical jacks (e.g., Ethernet, coaxial, or phone jacks) or one or more antennas to connect to the communications network 926. In an example, the network interface device 920 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 900, and includes digital or analog communications signals or other intangible medium to facilitate communication of such software.

The techniques described herein are not limited to any particular hardware or software configuration; they may find applicability in any computing, consumer electronics, or processing environment. The techniques may be implemented in hardware, software, firmware or a combination, resulting in logic or circuitry which supports execution or performance of embodiments described herein.

For simulations, program code may represent hardware using a hardware description language or another functional description language which essentially provides a model of how designed hardware is expected to perform. Program code may be assembly or machine language, or data that may be compiled or interpreted. Furthermore, it is common in the art to speak of software, in one form or another as taking an action or causing a result. Such expressions are merely a shorthand way of stating execution of program code by a processing system which causes a processor to perform an action or produce a result.

Each program may be implemented in a high-level procedural, declarative, or obj ect-oriented programming language to communicate with a processing system. However, programs may be implemented in assembly or machine language, if desired. In any case, the language may be compiled or interpreted.

Program instructions may be used to cause a general-purpose or special-purpose processing system that is programmed with the instructions to perform the operations described herein. Alternatively, the operations may be performed by specific hardware components that contain hardwired logic for performing the operations, or by any combination of programmed computer components and custom hardware components. The methods described herein may be provided as a computer program product, also described as a computer or machine accessible or readable medium that may include one or more machine accessible storage media having stored thereon instructions that may be used to program a processing system or other electronic device to perform the methods.

Program code, or instructions, may be stored in, for example, volatile or non-volatile memory, such as storage devices or an associated machine readable or machine accessible medium including solid-state memory, hard-drives, floppy-disks, optical storage, tapes, flash memory, memory sticks, digital video disks, digital versatile discs (DVDs), etc., as well as more exotic mediums such as machine-accessible biological state preserving storage. A machine readable medium may include any mechanism for storing, transmitting, or receiving information in a form readable by a machine, and the medium may include a tangible medium through which electrical, optical, acoustical or other form of propagated signals or carrier wave encoding the program code may pass, such as antennas, optical fibers, communications interfaces, etc. Program code may be transmitted in the form of packets, serial data, parallel data, propagated signals, etc., and may be used in a compressed or encrypted format.

Program code may be implemented in programs executing on programmable machines such as mobile or stationary computers, personal digital assistants, smart phones, mobile Internet devices, set top boxes, cellular telephones and pagers, consumer electronics devices (including DVD players, personal video recorders, personal video players, satellite receivers, stereo receivers, cable TV receivers), and other electronic devices, each including a processor, volatile or non-volatile memory readable by the processor, at least one input device or one or more output devices. Program code may be applied to the data entered using the input device to perform the described embodiments and to generate output information. The output information may be applied to one or more output devices. One of ordinary skill in the art may appreciate that embodiments of the disclosed subject matter may be practiced with various computer system configurations, including multiprocessor or multiple-core processor systems, minicomputers, mainframe computers, as well as pervasive or miniature computers or processors that may be embedded into virtually any device. Embodiments of the disclosed subject matter may also be practiced in distributed computing environments, cloud environments, peer-to-peer or networked microservices, where tasks or portions thereof may be performed by remote processing devices that are linked through a communications network.

A processor subsystem may be used to execute the instruction on the machine-readable or machine accessible media. The processor subsystem may include one or more processors, each with one or more cores. Additionally, the processor subsystem may be disposed on one or more physical devices. The processor subsystem may include one or more specialized processors, such as a graphics processing unit (GPU), a digital signal processor (DSP), a field programmable gate array (FPGA), or a fixed function processor.

Although operations may be described as a sequential process, some of the operations may in fact be performed in parallel, concurrently, or in a distributed environment, and with program code stored locally or remotely for access by single or multi-processor machines. In addition, in some embodiments the order of operations may be rearranged without departing from the disclosed subject matter. Program code may be used by or in conjunction with embedded controllers.

Examples, as described herein, may include, or may operate on, circuitry, logic or a number of components, modules, or mechanisms. Modules may be hardware, software, or firmware communicatively coupled to one or more processors in order to carry out the operations described herein. It will be understood that the modules or logic may be implemented in a hardware component or device, software or firmware running on one or more processors, or a combination. The modules may be distinct and independent components integrated by sharing or passing data, or the modules may be subcomponents of a single module, or be split among several modules. The components may be processes running on, or implemented on, a single compute node or distributed among a plurality of compute nodes running in parallel, concurrently, sequentially or a combination, as described more fully in conjunction with the flow diagrams in the figures. As such, modules may be hardware modules, and as such modules may be considered tangible entities capable of performing specified operations and may be configured or arranged in a certain manner. In an example, circuits may be arranged (e.g., internally or with respect to external entities such as other circuits) in a specified manner as a module. In an example, the whole or part of one or more computer systems (e.g., a standalone, client or server computer system) or one or more hardware processors may be configured by firmware or software (e.g., instructions, an application portion, or an application) as a module that operates to perform specified operations. In an example, the software may reside on a machine-readable medium. In an example, the software, when executed by the underlying hardware of the module, causes the hardware to perform the specified operations. Accordingly, the term hardware module is understood to encompass a tangible entity, be that an entity that is physically constructed, specifically configured (e.g., hardwired), or temporarily (e.g., transitorily) configured (e.g., programmed) to operate in a specified manner or to perform part or all of any operation described herein. Considering examples in which modules are temporarily configured, each of the modules need not be instantiated at any one moment in time. For example, where the modules comprise a general-purpose hardware processor configured, arranged or adapted by using software; the general-purpose hardware processor may be configured as respective different modules at different times. Software may accordingly configure a hardware processor, for example, to constitute a particular module at one instance of time and to constitute a different module at a different instance of time. Modules may also be software or firmware modules, which operate to perform the methodologies described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to suggest a numerical order for their objects.

While this subject matter has been described with reference to illustrative embodiments, this description is not intended to be construed in a limiting or restrictive sense. For example, the above-described examples (or one or more aspects thereof) may be used in combination with others. Other embodiments may be used, such as will be understood by one of ordinary skill in the art upon reviewing the disclosure herein. The Abstract is to allow the reader to quickly discover the nature of the technical disclosure. However, the Abstract is submitted with the understanding that it will not be used to interpret or limit the scope or meaning of the claims.

## Claims

1. A computer-implemented method for mitigating neuro-muscular ailments, the method comprising:
measuring (821), using device sensors (211) of an assistive device (121), at least one of: motion, pressure, or contraction and relaxation of muscles of an extremity of a user on which the assistive device is arranged to be worn;
processing (822) sensor data to infer an intended motion of the user, the sensor data received from the device sensors of the assistive device, and environmental sensors, wherein the environmental sensors are arranged external to the assistive device or part of the assistive device, and wherein processing the sensor data includes using context information derived from the sensor data to infer the intended motion, wherein the context information include at least one of: high-probability situational context, high-probability operational context, and high-probability motion context, wherein using the derived context information is performed with a technique configured with a plurality of accuracy levels for different operational modes, wherein an accuracy level is dependent on available sensor data in a current context related to the intended motion, and available data in a historical context arranged to be used to refine the likelihood of what is going to occur given the current context; and
controlling (823), based on the inferred intended motion, actuators (127, 301) and mechanical elements of the assistive device to thereby enable augmentation of the muscle strength of the extremity to assist performance of the intended motion, which includes applying electrical current to the actuators to control the mechanical elements coupled thereto to at least one of initiate, promote and suppress muscle movement of the extremity to assist performance of the intended motion.

2. The method of claim 1, wherein the sensor data includes measurements of at least one of motion, an object, a gesture, speech, an audible sound other than speech, location, or proximity.

3. The method of any of claims 1-2, wherein controlling the actuators to achieve the intended motion via augmentation of the muscles of the user further includes modifying the control based on an operational mode, wherein the operational mode is one of: a passive-reactive mode, an active-reactive mode, an active-predictive mode, an override mode, or an exercise mode.

4. The method of claim 3, wherein the passive-reactive mode mitigates unintended motion, the active-reactive mode assists the user with loss of strength, the active-predictive mode predicts the intended motion, and the exercise mode is to promote strength and dexterity retention and to monitor current abilities of the user.

5. The method of any of claims 1-4, wherein the environmental sensors include at least one of: a microphone, accelerometer, gyroscope, global positioning system, GPS, sensor, proximity sensor, location sensor; compass, camera, or physiological sensor.

6. The method as recited in claim 1, wherein the context information is provided to a trained machine learning model to infer the intended motion for the user.

7. The method of claim 6, comprising, responsive to an audible command by the user made in response to control of the actuators, implementing an override mode that includes:
modifying the control of the actuators to comply with the audible command; and
retraining the trained machine learning model with a current context from the sensor data and the audible command to improve future inferences.

8. The method of claim 7, comprising sending an operational request to an object corresponding to a physical object profile that includes the custom modality, the operational request sent in response to the intended motion corresponding to operation of the object.

9. The method of any of claims 1-8, comprising:
storing, on a memory, physical object profiles, a physical object profile including at least one of a standard modality profile, an assisted modality profile, or a custom modality profile; and
creating the high-probability operational context from the physical object profiles.

10. The method of any of claims 1-9, wherein controlling the actuators further includes modifying the control based on current abilities of the user to adjust strength assistance levels.

11. The method of any of claims 1-10, wherein processing the sensor data to infer the intended motion for the user includes using context derived from the sensor data to infer the intended motion, the intended motion including one or more actions.

12. The method of claim 11, wherein analysis of the available sensor data and historical context is arranged to be distributed between first processing circuitry included in the assistive device and second processing circuitry that is remote from the assistive device, wherein the first processing circuitry is configured with access to a memory including object profiles familiar to the user, and the second processing circuitry is configured with access to a memory that includes object profiles for objects unfamiliar to the user and the historical context data, wherein the first processing circuitry is arranged to infer the intended motion when disconnected from the second processing circuitry at a lower accuracy level than when communicatively connected to the second processing circuitry.

13. At least one machine-readable medium including instructions that, when executed by a processing circuitry of an assistive device arranged with device sensors, environmental sensors, and actuators coupled to mechanical elements of the assistive device, cause the processing circuitry to perform the method of any of claims 1-12.

14. A system comprising respective means configured to perform the respective steps of the method of any of claims 1-12.

## Patentansprüche

1. Computerimplementiertes Verfahren zur Abschwächung von neuromuskulären Beschwerden, wobei das Verfahren umfasst:
unter Verwendung von Vorrichtungssensoren (211) einer Assistenzvorrichtung (121) Messen (821) von mindestens einem von: Bewegung, Druck, oder Kontraktion und Relaxation von Muskeln einer Extremität eines Benutzers, woran die Assistenzvorrichtung zum Tragen vorgesehen ist; Verarbeiten (822) von Sensordaten, um eine geplante Bewegung des Benutzers schlusszufolgern, wobei die Sensordaten von den Vorrichtungssensoren der Assistenzvorrichtung sowie Umweltsensoren empfangen werden, wobei die Umweltsensoren außerhalb der Assistenzvorrichtung oder Teil(en) der Assistenzvorrichtung vorgesehen sind, und wobei Verarbeiten der Sensordaten Verwenden von Kontextinformationen einschließt, die von den Sensordaten abgeleitet sind, um die geplante Bewegung schlusszufolgern, wobei die Kontextinformationen mindestens eines von: hochwahrscheinlichem Situationskontext, hochwahrscheinlichem operativem Kontext und hochwahrscheinlichem Bewegungskontext einschließen, wobei Verwenden der abgeleiteten Kontextinformationen mit einer Technik durchgeführt wird, die für unterschiedliche operative Modi mit einer Vielzahl von Genauigkeitsniveaus konfiguriert ist, wobei ein Genauigkeitsniveau von verfügbaren Sensordaten in einem aktuellen Kontext, der mit der geplanten Bewegung in Beziehung steht, sowie verfügbaren Daten in einem historischen Kontext abhängt, die vorgesehen sind, um zur Präzisierung der Wahrscheinlichkeit, was sich bei gegebenem aktuellen Kontext ereignen wird, verwendet zu werden; und
basierend auf der schlussgefolgerten geplanten Bewegung Steuern (823) von Aktuatoren (127, 301) und mechanischen Elementen der Assistenzvorrichtung, um dadurch Augmentation der Muskelstärke der Extremität zu ermöglichen, um Ausführung der geplanten Bewegung zu assistieren, was Anlegen von elektrischem Strom an die Aktuatoren einschließt, um die daran gekoppelten mechanischen Elemente zu mindestens einem von Initiieren, Fördern und Unterdrücken der Muskelbewegung der Extremität zur Assistenz der Ausübung der geplanten Bewegung zu steuern.

2. Verfahren nach Anspruch 1, wobei die Sensordaten Messungen von mindestens einem von Bewegung, einem Objekt, einer Geste, Sprache, einem von Sprache verschiedenen hörbaren Klang, Ort oder Nähe einschließen.

3. Verfahren nach einem der Ansprüche 1-2, wobei Steuern der Aktuatoren, um die geplante Bewegung über Augmentation der Muskeln des Benutzers zu erreichen, des Weiteren Modifizieren der Steuerung basierend auf einem operativen Modus einschließt, wobei der operative Modus einer von: einem passiv-reaktiven Modus, einem aktivreaktiven Modus, einem aktiv-prädiktiven Modus, einem Überschreibemodus oder einem Übungsmodus ist.

4. Verfahren nach Anspruch 3, wobei der passiv-reaktive Modus ungeplante Bewegung abschwächt, der aktiv-reaktive Modus dem Benutzer mit Kraftverlust assistiert, der aktiv-prädiktive Modus die geplante Bewegung vorhersagt, und der Übungsmodus zur Förderung von Kraft und Bewahrung der Geschicklichkeit und zur Überwachung der aktuellen Fähigkeiten des Benutzers dient.

5. Verfahren nach einem der Ansprüche 1-4, wobei die Umweltsensoren mindestens einen von: einem Mikrofon, Beschleunigungsmesser, Gyroskop, Global Positioning System, GPS, Sensor, Näherungssensor, Ortungssensor; Kompass, Kamera oder physiologischem Sensor einschließen.

6. Verfahren nach Anspruch 1, wobei die Kontextinformationen einem trainierten maschinellen Lernmodell bereitgestellt werden, um die geplante Bewegung für den Benutzer schlusszufolgern.

7. Verfahren nach Anspruch 6, umfassend in Reaktion auf einen hörbaren Befehl seitens des Benutzers, der in Reaktion auf Steuerung der Aktuatoren erfolgte, Implementieren eines Überschreibemodus, der einschließt: Modifizieren der Steuerung der Aktuatoren, um dem hörbaren Befehl zu entsprechen; und Retraining des trainierten maschinellen Lernmodells mit einem aktuellen Kontext aus den Sensordaten und dem hörbaren Befehl, um zukünftige Schlussfolgerungen zu verbessern.

8. Verfahren nach Anspruch 7, umfassend Senden einer operativen Anfrage an ein Objekt entsprechend einem physischen Objektprofil, welches die individuelle Modalität einschließt, wobei die operative Anfrage in Reaktion auf die geplante Bewegung gesendet wird, die Operation des Objekts entspricht.

9. Verfahren nach einem der Ansprüche 1-8, umfassend: Speichern von physischen Objektprofilen in einem Speicher, wobei ein physisches Objektprofil mindestens eines von einem Standardmodalitätsprofil, einem assistierten Modalitätsprofil oder einem individuellen Modalitätsprofil einschließt; und Erstellen des hochwahrscheinlichen operativen Kontextes aus den physischen Objektprofilen.

10. Verfahren nach einem der Ansprüche 1-9, wobei Steuern der Aktuatoren des Weiteren Modifizieren der Steuerung basierend auf aktuellen Fähigkeiten des Benutzers einschließt, um Kraftassistenzniveaus anzupassen.

11. Verfahren nach einem der Ansprüche 1-10, wobei Verarbeiten der Sensordaten zum Schlussfolgern der geplanten Bewegung für den Benutzer Verwenden von Kontext einschließt, der von den Sensordaten abgeleitet ist, um die geplante Bewegung schlusszufolgern, wobei die geplante Bewegung eine oder mehrere Aktionen einschließt.

12. Verfahren nach Anspruch 11, wobei Analyse der verfügbaren Sensordaten und des historischen Kontextes so vorgesehen ist, dass sie zwischen ersten Verarbeitungsschaltung(en), die in die Assistenzvorrichtung eingeschlossen ist/sind, und zweiten Verarbeitungsschaltung(en), die sich entfernt von der Assistenzvorrichtung befindet/befinden, aufgeteilt wird, wobei die erste(n) Verarbeitungsschaltung(en) mit Zugang zu einem Speicher konfiguriert ist/sind, der Objektprofile einschließt, die dem Benutzer vertraut sind, und die zweite(n) Verarbeitungsschaltung(en) mit Zugang zu einem Speicher konfiguriert ist/sind, der Objektprofile für Objekte, die dem Benutzer nicht vertraut sind, und die historischen Kontextdaten einschließt, wobei die erste(n) Verarbeitungsschaltung(en) vorgesehen ist/sind, um die geplante Bewegung, wenn keine Verbindung zu der/den zweite(n) Verarbeitungsschaltung(en) besteht, mit einem geringeren Genauigkeitsniveau schlusszufolgern, als wenn kommunikative Verbindung zu der/den zweite(n) Verarbeitungsschaltung(en) besteht.

13. Mindestens ein maschinenlesbares Medium, das Anweisungen einschließt, die bei Ausführung durch Verarbeitungsschaltung(en) einer Assistenzvorrichtung, bei der Vorrichtungssensoren, Umweltsensoren und Aktuatoren, die an mechanische Elemente der Assistenzvorrichtung gekoppelt sind, vorgesehen sind, bewirken, dass die Verarbeitungsschaltung(en) das Verfahren gemäß einem der Ansprüche 1-12 durchführt/durchführen.

14. System, umfassend jeweilige Mittel, die konfiguriert sind, um die jeweiligen Schritte des Verfahrens gemäß einem der Ansprüche 1-12 auszuführen.

## Revendications

1. Procédé mis en oeuvre par ordinateur pour atténuer des affections neuro-musculaires, le procédé comprenant :
la mesure (821), à l'aide de capteurs de dispositif (211) d'un dispositif d'assistance (121), d'au moins l'un parmi : un mouvement, une pression, ou une contraction et une relaxation des muscles d'une extrémité d'un utilisateur sur laquelle le dispositif d'assistance est agencé pour être porté ;
le traitement (822) de données de capteur pour déduire un mouvement intentionnel de l'utilisateur, les données de capteur étant reçues des capteurs de dispositif du dispositif d'assistance et de capteurs environnementaux, les capteurs environnementaux étant agencés à l'extérieur du dispositif d'assistance ou d'une partie du dispositif d'assistance, et le traitement des données de capteur comprenant l'utilisation d'informations contextuelles dérivées des données de capteur pour déduire le mouvement intentionnel, les informations contextuelles comprenant au moins l'un des éléments suivants : un contexte situationnel à forte probabilité, un contexte opérationnel à forte probabilité et un contexte de mouvement à forte probabilité, l'utilisation des informations contextuelles dérivées étant réalisée à l'aide d'une technique configurée avec une pluralité de niveaux de précision pour différents modes opérationnels, un niveau de précision dépendant des données de capteur disponibles dans un contexte actuel lié au mouvement intentionnel et des données disponibles dans un contexte historique agencé pour être utilisé afin d'affiner la probabilité de ce qui va se produire compte tenu du contexte actuel ; et
la commande (823), sur la base du mouvement intentionnel déduit, d'actionneurs (127, 301) et d'éléments mécaniques du dispositif d'assistance pour permettre ainsi l'augmentation de la force musculaire de l'extrémité afin d'aider à la réalisation du mouvement intentionnel, ce qui comprend l'application d'un courant électrique aux actionneurs pour commander les éléments mécaniques couplés à ceux-ci pour au moins l'un des éléments suivants : initier, promouvoir et supprimer le mouvement musculaire de l'extrémité afin d'aider à la réalisation du mouvement intentionnel.

2. Procédé selon la revendication 1, les données de capteur comprenant des mesures d'au moins un élément parmi un mouvement, un objet, un geste, la parole, un son audible autre que la parole, l'emplacement ou la proximité.

3. Procédé selon l'une quelconque des revendications 1 et 2, la commande des actionneurs pour réaliser le mouvement intentionnel par l'intermédiaire de l'augmentation des muscles de l'utilisateur comprenant en outre la modification de la commande sur la base d'un mode opérationnel, le mode opérationnel étant l'un des suivants : un mode passif-réactif, un mode actif-réactif, un mode actif-prédictif, un mode prioritaire ou un mode d'exercice.

4. Procédé selon la revendication 3, le mode passif-réactif atténuant un mouvement involontaire, le mode actif-réactif assiste l'utilisateur en cas de perte de force, le mode actif-prédictif prédisant le mouvement intentionnel, et le mode d'exercice étant destiné à promouvoir le maintien de la force et de la dextérité et à surveiller les capacités actuelles de l'utilisateur.

5. Procédé selon l'une quelconque des revendications 1 à 4, les capteurs environnementaux comprenant au moins l'un des éléments suivants : un microphone, un accéléromètre, un gyroscope, un capteur de système de positionnement global, GPS, un capteur de proximité, un capteur de localisation, une boussole, une caméra ou un capteur physiologique.

6. Procédé selon la revendication 1, les informations contextuelles étant fournies à un modèle d'apprentissage automatique formé pour déduire le mouvement intentionnel pour l'utilisateur.

7. Procédé selon la revendication 6, comprenant, en réponse à une commande audible de l'utilisateur faite en réponse à la commande des actionneurs, la mise en oeuvre d'un mode prioritaire qui comprend : la modification de la commande des actionneurs pour se conformer à la commande audible ; et le réentraînement du modèle d'apprentissage automatique formé avec un contexte actuel à partir des données de capteur et de la commande audible pour améliorer des déductions futures.

8. Procédé selon la revendication 7, comprenant l'envoi d'une demande opérationnelle à un objet correspondant à un profil d'objet physique qui inclut la modalité personnalisée, la demande opérationnelle étant envoyée en réponse au mouvement intentionnel correspondant à l'opération de l'objet.

9. Procédé selon l'une quelconque des revendications 1 à 8, comprenant :
le stockage, sur une mémoire, de profils d'objets physiques, un profil d'objet physique comprenant au moins un profil de modalité standard, un profil de modalité assistée ou un profil de modalité personnalisée ; et la création du contexte opérationnel à haute probabilité à partir des profils d'objets physiques.

10. Procédé selon l'une quelconque des revendications 1 à 9, la commande des actionneurs comprenant en outre la modification de la commande sur la base des capacités actuelles de l'utilisateur pour ajuster les niveaux d'assistance de la force.

11. Procédé selon l'une quelconque des revendications 1 à 10, le traitement des données de capteur pour déduire le mouvement intentionnel pour l'utilisateur comprenant l'utilisation du contexte dérivé des données de capteur pour déduire le mouvement intentionnel, le mouvement intentionnel comprenant une ou plusieurs actions.

12. Procédé selon la revendication 11, l'analyse des données de capteur disponibles et du contexte historique étant agencée pour être distribuée entre un premier circuit de traitement inclus dans le dispositif d'assistance et un second circuit de traitement qui est distant du dispositif d'assistance, le premier circuit de traitement étant configuré avec un accès à une mémoire comprenant des profils d'objets familiers à l'utilisateur, et le second circuit de traitement étant configuré pour accéder à une mémoire comprenant des profils d'objets non familiers à l'utilisateur et des données contextuelles historiques, le premier circuit de traitement étant agencé pour déduire le mouvement intentionnel lorsqu'il est déconnecté du second circuit de traitement avec un niveau de précision inférieur à celui qu'il a lorsqu'il est connecté de manière communicative au second circuit de traitement.

13. Au moins un support lisible par machine comprenant des instructions qui, lorsqu'elles sont exécutées par un circuit de traitement d'un dispositif d'assistance agencé avec des capteurs de dispositif, des capteurs environnementaux et des actionneurs couplés à des éléments mécaniques du dispositif d'assistance, amènent le circuit de traitement à réaliser le procédé selon l'une quelconque des revendications 1 à 12.

14. Système comprenant des moyens respectifs configurés pour réaliser les étapes respectives du procédé selon l'une quelconque des revendications 1 à 12.
